(19) Europäisches Patentamt
European Patent Office
Office européen des brevets

(11) EP 3 322 821 B1

(12) **FASCICULE DE BREVET EUROPEEN**

(45) Date de publication et mention de la délivrance du brevet:
**24.11.2021 Bulletin 2021/47**

(21) Numéro de dépôt: **16750971.0**

(22) Date de dépôt: **18.07.2016**

(51) Int Cl.:
***C12Q 1/6886*** *(2018.01)* ***C12Q 1/6883*** *(2018.01)*

(86) Numéro de dépôt international:
**PCT/FR2016/051838**

(87) Numéro de publication internationale:
**WO 2017/009588 (19.01.2017 Gazette 2017/03)**

(54) **METHODE DE PRONOSTIC DE PATHOLOGIES**

PROGNOSEVERFAHREN FÜR PATHOLOGIEN

METHOD OF PROGNOSIS OF PATHOLOGIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.07.2015 FR 1556731**

(43) Date de publication de la demande:
**23.05.2018 Bulletin 2018/21**

(73) Titulaires:
- **Centre National de la Recherche Scientifique CNRS**
  **75794 Paris Cedex 16 (FR)**
- **Université de Montpellier**
  **34090 Montpellier (FR)**
- **AxLR, SATT du Languedoc Roussillon**
  **34095 Montpellier (FR)**
- **Institut National de la Santé et de la Recherche Médicale (INSERM)**
  **75013 Paris (FR)**

(72) Inventeur: **BIENVENU, Frédéric**
**34980 Saint-Clément-de-Rivière (FR)**

(74) Mandataire: **LLR**
**11 boulevard de Sébastopol**
**75001 Paris (FR)**

(56) Documents cités:
**WO-A1-2006/059952**

- **ALMENDRAL J M ET AL: "CLONING AND SEQUENCE OF THE HUMAN NUCLEAR PROTEIN CYCLIN: HOMOLOGY WITH DNA-BINDING PROTEINS", PROCEEDINGS OF THE NATIONAL ACADEMY OF SCIENCES, NATIONAL ACADEMY OF SCIENCES, US, vol. 84, 1 mars 1987 (1987-03-01), pages 1575-1579, XP002952108, ISSN: 0027-8424, DOI: 10.1073/PNAS.84.6.1575**
- **VIEIRA J ET AL: "The pUC plasmids, an M13mp7-derived system for insertion mutagenesis and sequencing with synthetic universal primers", GENE, ELSEVIER, AMSTERDAM, NL, vol. 19, no. 3, 1 octobre 1982 (1982-10-01), pages 259-268, XP023539775, ISSN: 0378-1119, DOI: 10.1016/0378-1119(82)90015-4 [extrait le 1982-10-01]**
- **TANIOKA M ET AL: "Transcriptional CCND1 expression as a predictor of poor response to neoadjuvant chemotherapy with trastuzumab in HER2-positive/ER-positive breast cancer", BREAST CANCER RESEARCH AND TREATMENT, SPRINGER , NY, US, vol. 147, no. 3, 9 septembre 2014 (2014-09-09), pages 513-525, XP035396750, ISSN: 0167-6806, DOI: 10.1007/S10549-014-3121-5 [extrait le 2014-09-09]**
- **ASHTON KATIE A ET AL: "The influence of the Cyclin D1 870 G>A polymorphism as an endometrial cancer risk factor", BMC CANCER, BIOMED CENTRAL, LONDON, GB, vol. 8, no. 1, 29 septembre 2008 (2008-09-29), page 272, XP021042910, ISSN: 1471-2407, DOI: 10.1186/1471-2407-8-272**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **CHENG GANG ET AL: "Overexpression of cyclin D1 in meningioma is associated with malignancy grade and causes abnormalities in apoptosis, invasion and cell cycle progression", MEDICAL ONCOLOGY, SCIENCE AND TECHNOLOGY LETTERS, NORTHWOOD, GB, vol. 32, no. 1, 13 décembre 2014 (2014-12-13), pages 1-8, XP035430305, ISSN: 1357-0560, DOI: 10.1007/S12032-014-0439-0 [extrait le 2014-12-13]**
- **YONG GUO ET AL: "Let-7b expression determines response to chemotherapy through the regulation of Cyclin D1 in Glioblastoma", JOURNAL OF EXPERIMENTAL & CLINICAL CANCER RESEARCH, BIOMED CENTRAL LTD, LONDON UK, vol. 32, no. 1, 27 juin 2013 (2013-06-27), page 41, XP021157291, ISSN: 1756-9966, DOI: 10.1186/1756-9966-32-41**
- **MAIMOONA SABIR ET AL: "Significance of Cyclin D1 Polymorphisms in Patients with Head and Neck Cancer", THE INTERNATIONAL JOURNAL OF BIOLOGICAL MARKERS ITALY, vol. 28, no. 1, 1 January 2013 (2013-01-01), pages 49-55, XP055591649, ISSN: 1724-6008, DOI: 10.5301/JBM.2012.9768**

## Description

La présente invention concerne une méthode de pronostic de pathologies.

La Cycline D1 est un des composants de la machinerie du cycle cellulaire durant le développement, mais semble ne pas être indispensable lors de la vie adulte.

Durant le développement, les Cyclines de type D, (Cyclines D1, D2 et D3) sont au cœur de l'intégration des signaux mitogéniques extracellulaires. Pour orchestrer la phase G1 du cycle cellulaire et soutenir l'entrée en phase S, les Cyclines D peuvent promouvoir la phosphorylation de RB ou les protéines de la famille RB (pocket proteins en anglais) par l'intermédiaire de l'activation des kinases dépendantes des Cyclines CDK4/6, peuvent titrer les régulateurs du cycle cellulaire de la famille Cdkn1 pour permettre le relargage de l'activité Cyclines E/CDK2, et peuvent influencer la transcription de gènes en interagissant avec les promoteurs.

Du fait de la plasticité du cycle cellulaire, l'invalidation des trois gènes codant les Cyclines D n'inhibe pas formellement la division cellulaire. En effet, la plupart des Cyclines impliquées dans le cycle cellulaire présentent des activités redondantes, mais l'important est l'expression tissulaire. Aussi, l'invalidation d'un seul gène codant une des trois Cyclines D empêche un développement correct et complet.

De manière intéressante, le gène *Ccnd1,* qui code la Cycline D1 est le second gène le plus fortement altéré dans les cancers chez l'homme. En effet, on retrouve une forte expression de la Cycline D1 dans une majorité de cancers et notamment les carcinomes mammaires, ce qui renforce la fonction oncogénique du gène *Ccnd1.* Aussi, la Cycline D1 devient une cible thérapeutique privilégiée pour traiter le cancer.

En effet, les études réalisées chez les souris invalidées pour le gène *Ccnd1* (, (*Ccnd1-/-* n'exprimant pas de Cycline D1) ont montré que les femelles sont protégées contre les tumeurs du sein induites par les oncogènes ErbB2 ou Ras, probablement du fait de l'absence d'activité CDK4.

L'invalidation conditionnelle de la Cycline D1 chez les souris adultes suggère son implication dans le développement des tumeurs dépendantes de ErbB2.

Toutefois, l'absence d'activation de CDK4 par la Cycline D1 conduit à une diminution de cellules mammaires progénitrices chez les souris adultes après plusieurs gestations.

Aussi, pour déterminer si la Cycline D1 est un véritable candidat en tant que cible thérapeutique du cancer, il reste à savoir si la Cycline D1 a des fonctions dans les organes adultes et si l'invalidation de la Cycline D1 a un potentiel thérapeutique dans les tumeurs. La même remarque s'applique aux Cyclines D2 et D3.

En particulier, l'impact de la Cycline D1 dans le pronostic de réponse aux traitements des patients cancéreux reste à déterminer.

Un des buts de l'invention est de résoudre ce problème.

Un autre but de l'invention est de proposer une méthode de pronostic fiable de la susceptibilité aux différents stress environnementaux.

Encore un autre but de l'invention est de fournir des traitements adaptés à certaines tumeurs impliquant la Cycline D1.

Aussi, l'invention concerne une méthode de pronostic, notamment *in vitro,* de la susceptibilité à un stress environnemental d'un individu, la méthode comprenant :

a. une étape de détermination de la séquence d'initiation de la traduction, ou séquence KOZAK, de l'un au moins des gènes de la famille des Cylines de type *D, Ccnd1, Ccnd2, Ccnd3, Ccne1 et Ccne2,* à partir d'un échantillon biologique dudit individu, et la comparaison avec une séquence correspondante de référence pour identifier des mutations,

b. une étape de quantification de l'expression de la protéine codée par ledit gène dont la séquence KOZAK a été séquencée à l'étape précédente, et la comparaison avec le niveau d'expression de ladite protéine issue d'un échantillon de référence,

de sorte que si la séquence KOZAK comprend une mutation et que le niveau d'expression de la protéine est modifié par rapport au niveau de référence, ledit individu sera plus susceptible de développer une pathologie liée à un stress environnemental.

L'invention repose sur la constatation surprenante faite par les inventeurs selon laquelle la quantité de Cycline D1 influence la réponse cellulaire à un stress environnemental. En outre, les inventeurs ont identifié que certaines mutations dans la séquence de l'ARN messager de la Cycline D1, la Cycline D2 ou la Cycline D3, ou encore la Cycline E1 ou la Cycline E2 pouvaient influer sur la traduction de la protéine. En d'autres termes, il existe des mutations dans la séquence du gène codant la Cycline D1, ou de celui codant la Cycline D2 ou celui codant la Cycline D3, ou codant la Cycline E1 ou codant la Cycline E2, qui ne modifient pas le niveau d'expression des ARN messagers, n'altèrent pas la fonction de la protéine, mais interfèrent avec la traduction protéique et donc la quantité finale de protéine.

On entend par stress environnemental l'exposition à une condition environnementale délétère ou à une substance toxique. Le stress environnemental ayant pour conséquence de mettre en péril l'intégrité cellulaire au risque de conduire à la mort cellulaire, par le biais par exemple de cassures de l'ADN, de génération d'entité réactives oxidantes ou encore d'une perturbation du potentiel des membranes.

Ces mutations, qui interfèrent avec la traduction en protéine des ARN messagers de Cycline D1, Cycline D2 et Cycline D3, et les cyclines E1 et E2 sont des mutations localisées dans la séquence d'initiation de la traduction, également appelée séquence KOZAK ou Kozak. La séquence Kozak est une séquence conservée que l'on trouve sur les ARN messagers eucaryotes au niveau du site de démarrage de la traduction, autour du codon de démarrage ATG (ou AUG si le codon est lu sur une séquence d'ARN). Chez les vertébrés, la séquence de Kozak a pour consensus gccRcc<u>AUG</u>G où R représente une purine (le codon de démarrage est souligné et les nucléotides en minuscules sont moins conservés que le reste de la séquence).

Il est montré que les mutations dans cette région, qui peuvent être des substitutions (c'est à dire le changement d'un nucléotide par un autre), des insertions d'au moins un nucléotide ou des délétions d'au moins un nucléotide, peuvent avoir un effet positif ou négatif sur la traduction.

Par effet positif sur la traduction, on parlera d'une mutation qui a pour effet d'augmenter la traduction par rapport à la même séquence ne possédant pas la mutation, l'augmentation étant d'au moins 10%. Dès lors, on considèrera qu'une mutation a un effet positif si sa présence dans une séquence Kozak conduit à une augmentation de 15% de la quantité de protéine par rapport à la quantité de protéine obtenue avec la séquence Kozak ne possédant pas ladite mutation.

Par effet négatif sur la traduction, on parlera d'une mutation qui a pour effet de diminuer la traduction par rapport à la même séquence ne possédant pas la mutation, la diminution étant d'au moins 10%. Dès lors, on considèrera qu'une mutation a un effet négatif si sa présence dans une séquence Kozak conduit à une diminution de 15% de la quantité de protéine par rapport à la quantité de protéine obtenue avec la séquence Kozak ne possédant pas ladite mutation.

Au vu de ces éléments on comprend que la méthode de l'invention nécessite deux étapes :

- une étape de caractérisation de la mutation, c'est à dire une étape qui identifie la différence de séquence par rapport à la séquence de référence, et
- une étape de mesure du niveau d'expression de protéines codées par des molécules d'acide nucléique possédant ladite mutation, par rapport au niveau protéique de la même molécule d'acides nucléiques ne possédant pas ladite mutation.

Etape de caractérisation de la mutation.

L'étape de caractérisation de la mutation dans une séquence Kozak d'un ARN messager codant une protéine de la famille des Cyclines D est une technique aisée pour l'homme du métier.

Parmi toutes les méthodes connues à ce jour, l'homme du métier peut utiliser

- La technique de SSCP, ou polymorphisme de conformation des simples brins (acronyme de l'anglais single strand conformation polymorphism) : il s'agit d'une technique visant à séparer différents allèles d'un même gène (allozyme) en misant sur la différence de migration dans un gel non dénaturant de leurs différentes conformations,
- la technique DGGE ou électrophorèse sur gel en gradient dénaturant (de l'anglais *denaturing gradient gel electrophoresis*) : il s'agit d'une technique d'électrophorèse permettant la séparation de molécules d'acides nucléiques (ADN ou ARN) de même taille. Son principe consiste à déposer un échantillon d'acide nucléique sur un gel d'électrophorèse contenant un agent dénaturant. Dans un gel DGGE, les fragments d'acide nucléique sont soumis à différentes concentrations croissantes en dénaturant. Les 2 brins d'ADN se séparent plus ou moins rapidement en fonction de leur composition en bases AT et GC (2 liaisons hydrogènes pour AT contre 3 pour GC). Deux molécules différentes peuvent avoir des brins qui ne se sépareront pas au même moment et migreront alors différemment. La molécule la plus stable migrera moins vite que celle qui se dénaturera dans le gradient.
- la technique FAMA (de l'anglais fluorescence-assisted mismatch analysis), dérivée de la technique de clivage chimique qui, en objectivant les mésappariements de doubles brins d'ADN hétérologues (hétéroduplexes) marqués à leurs extrémités par des fluorophores distincts, permet une exploration rapide d'un gène,
- ou encore la réaction de polymérisation en chaine, ou PCR, associée au séquençage.

L'homme du métier, selon les circonstances, sera capable de déterminer la méthode la plus appropriée, même si la méthode la plus avantageuse est la technique de PCR couplée au séquençage.

Dans le cadre de la technique de PCR couplée au séquençage, l'homme du métier déterminera aisément un couple d'oligonucléotides encadrant la région à amplifier (entourant la séquence Kozak dans laquelle il est suspecté la présence d'une mutation) et au moins un nucléotide permettant le séquençage de la région amplifiée. Les techniques de séquences sont très bien connues de l'état de la technique, et il n'est pas nécessaire de l'exposée ici plus avant.

Une fois la séquence obtenue, celle-ci est comparée à une séquence de référence qui est une séquence ne

comportant pas de mutation. La séquence de référence correspond à la séquence retrouvée majoritairement dans la population générale, dans la majorité des individus sains, ou n'étant pas porteurs de la maladie dont le pronostic ou le diagnostic est recherché.

Si les deux séquences sont identiques, dans ce cas il sera considéré que la séquence étudiée n'est pas une séquence mutée. La suite de la méthode ne sera donc pas appliquée. Si par contre la séquence étudiée diffère d'au moins un nucléotide par rapport à la séquence de référence, dans ce cas il sera nécessaire de déterminer si cette séquence dite mutée conduit à l'expression d'une quantité de protéine différente, par rapport à la quantité de protéine obtenue avec la séquence de référence, non mutée.

Etape de mesure du niveau d'expression de la protéine en aval de la séquence Kozak mutée.

Afin de déterminer si la séquence mutée a un effet positif ou un effet négatif sur la traduction, il est ensuite nécessaire de mesurer le niveau d'expression protéique d'une protéine placée en aval, ou en position 3', de la séquence Kozak dans laquelle une mutation a été trouvée.

Plusieurs méthodes peuvent être envisagées :

- il est possible par exemple de cloner la séquence mutée en amont d'une protéine reportrice, dont l'expression pourra être mesurée, et quantifiée de manière simple. Par exemple, on envisage comme protéine reportrice, une molécule autofluorescente de la famille des protéines fluorescentes, telle que la GFP, la RFP, la CFP ou la YFP et leurs dérivés, la protéine de luciole luciférase, une protéine présentant au moins une étiquette de type MYC, HA, HIS, V5, VSVG etc...,
- ou de mesurer directement l'expression de la protéine de la famille des Cyclines D ou E1 ou E2 dont le gène présente la mutation dans la séquence Kozak identifiée à l'étape précédente (voir par exemple l'Exemple 2).

Dans le premier exemple ci-dessus, la mesure de l'expression et de la quantification se fera selon le type de protéine exprimée sous contrôle de la séquence Kozak mutée. Par exemple, pour les protéines fluorescentes, le niveau d'expression pourra être mesuré par cytométrie de flux, dans le cas de la luciférase, le niveau d'expression pourra être mesuré par spectro-photométrie. Pour les protéines étiquetées une détection par immunoblot (ou western blot) est envisagée, ou la technique exposée ci-après.

Dans le cas de la mesure directe de l'expression des protéines de la famille Cycline D, une détection peut être réalisée par immunoblot, comme pour les protéines étiquetées, ou encore par TR-FRET.

En effet, les inventeurs ont montré que si l'on utilise deux anticorps dirigés contre la Cycline D1 et qui sont susceptibles de permettre le FRET, il est possible de quantifier la quantité de protéine Cycline D1 de manière fiable, spécifique et reproductible.

La méthode TR-FRET utilise au moins un couple d'anticorps (c'est-à-dire au moins deux anticorps) dirigés contre la même protéine Cycline D1, chacun des anticorps étant couplé à une molécule luminescente. La luminescence peut se définir comme une émission de lumière (photons) par une molécule luminescente (fluorophore, enzyme, etc.). Parmi les phénomènes de luminescence, on distingue la photoluminescence (fluorescence, phosphorescence) qui est consécutive à une excitation lumineuse, la chimioluminescence dont l'émission de lumière est consécutive à une réaction chimique et la bioluminescence qui est déclenché par une réaction enzymatique.

Lorsque deux molécules luminescentes (appelées respectivement le donneur et l'accepteur d'énergie) sont à proximité, il peut se produire un transfert d'énergie (RET) du donneur vers l'accepteur. Ce phénomène se traduit par l'extinction totale de la luminescence (fluorescence, bioluminescence, chimioluminescence) du donneur et par l'apparition d'une émission de fluorescence de l'accepteur. Ce transfert d'énergie s'effectue sans émission de lumière.

Pour cette raison, et afin de réaliser un transfert d'énergie entre la première molécule luminescente et la seconde molécule luminescente, le premier anticorps est appelé un anticorps donneur car il porte une première molécule luminescente qui, après excitation, transmettra son énergie à la seconde molécule luminescente portée par l'anticorps que l'on appellera anticorps accepteur.

Il est avantageux dans le cadre de la méthode de l'invention que lesdits anticorps donneur et accepteur reconnaissent chacun spécifiquement un épitope différent de ladite protéine codée par le gène CCND1, afin qu'ils puissent concomitamment interagir avec ladite protéine. En effet, pour obtenir du FRET (ou un transfert d'énergie) il est nécessaire que les molécules luminescentes soit proches spatialement.

Aussi, de manière avantageuse, les anticorps donneur et accepteur sont capables d'interagir avec des épitopes différents, qui sont localisés sur la même protéine de sorte que les molécules luminescentes qu'ils portent soient spécialement proches.

Dans l'invention, il est précisé que « lesdites première et deuxième molécules luminescentes sont telles que la longueur d'onde d'émission de la première molécule luminescente correspond à la longueur d'onde d'excitation de la deuxième molécule luminescente ». Cela signifie qu'en présence de deux molécules luminescentes (notamment des fluorophores) donneuse - accepteuse d'énergie, le phénomène d'excitation du donneur suit le processus décrit précédemment mais sa désexcitation peut, sous certaines conditions, passer par un transfert d'énergie non radiatif sur l'accepteur (et non par l'émission directe d'un photon). L'énergie de désexcitation du fluorophore donneur est donc « absorbée » par le fluorophore accepteur qui passe alors d'un état fondamental à un état excité. Le retour vers l'état fondamental de l'accepteur peut se faire par une émission d'un photon dont la longueur d'onde est plus grande que celle du photon qui aurait été émis par le fluorophore donneur en absence d'accepteur.

Les anticorps avantageux de l'invention sont

- l'anticorps anti Cycline D1 clone ab1, commercialisé par Thermofisher Scientific. Cet anticorps correspond à un anticorps monoclonal de souris de type $IgG_{2a/k}$ de souris aussi appelé clone DCS-6, obtenu par immunisation de souris avec la protéine Cycline D1 humaine entière,
- l'anticorps anti Cycline D1 clone ab3, commercialisé par Thermofisher Scientific. Il s'agit d'un anticorps polyclonal de lapin obtenu après immunisation avec la partie C-terminale de la protéine Cycline D1 humaine.
- l'anticorps anti Cycline D1 clone SP4, commercialisé par Life Technologies qui reconnait l'épitope C-terminal de la protéine Cycline D1 humaine, et dirigé contre le peptide : QIEALLESSLRQAQQNMDPKAAEEEEEEEEEVDLACTPTDVRDVDI (SEQ ID NO: 4), et
- l'anticorps anti Cycline D1 clone 72-13G, commercialisé par Santa Cruz Biotechnology Inc sous la référence sc450. Cet anticorps est anticorps monoclonal de souris dirigé contre une protéine de fusion Cycline D1 murine.

Comme cela a été mentionné ci-dessus, la méthode selon l'invention utilise un anticorps donneur et au moins un anticorps accepteur. Aussi, au vu des quatre anticorps avantageux susmentionnés, la méthode selon l'invention couvre les différentes 28 combinaisons suivantes :

| Donneur | Accepteur | Donneur | Accepteur | Donneur | Accepteur |
|---|---|---|---|---|---|
| ab1 | ab3 | ab3 | ab1 | SP4 | ab1 |
| | SP4 | | SP4 | | ab3 |
| | SC450 | | SC450 | | SC450 |
| | ab3 +SP4 | | ab1 +SP4 | | ab1 +ab3 |
| | ab3 +SC450 | | ab1 +SC450 | | ab1 +SC450 |
| | SP4 +SC450 | | SP4 +SC450 | | ab3 +SC450 |
| | ab3 +SP4 +SC450 | | ab1 +SP4 +SC450 | | ab1 +ab3 +SC450 |

| Donneur | Accepteur |
|---|---|
| sc450 | ab1 |
| | ab3 |
| | SP4 |
| | ab1 +ab3 |
| | ab1 +SP4 |
| | ab3+ SP4 |
| | ab1 +ab3 +SP4 |

Une des combinaisons avantageuses est la combinaison SC450 donneur et ab1+ab3 accepteur.

Dans le cadre du transfert d'énergie entre deux molécules luminescentes, ce qui est important c'est de disposer de molécules dont le spectre d'excitation soit le moins chevauchant avec son spectre d'émission. En d'autres termes, il est important pour une même molécule luminescente que le spectre d'émission et le spectre d'excitation soit le moins chevauchant possible, de sorte que la longueur d'onde d'excitation ne puisse pas correspondre à la longueur d'onde d'excitation d'une seconde molécule luminescente qui devrait être activée par transfert d'énergie. Dans le cas de chevauchement, il y aura un manque de spécificité spectral qui sera à l'origine de bruit de fond important ce qui diminuera largement la sensibilité de la quantification.

Le développement de molécules luminescentes avec des propriétés singulières a permis d'améliorer considérablement la sélectivité spectrale des couples de fluorophores organiques. Les propriétés de ces fluorophores permettent d'effectuer des mesures de FRET dans des intervalles de temps longs (de plusieurs microsecondes à la milliseconde contre quelques nanosecondes pour des fluorophores classiques). C'est pourquoi on utilise le terme de temps résolu (Time - Resolved, TR) ou sélectivité temporelle pour les techniques de RET utilisant ces molécules.

Les cryptates de terre rare sont des complexes formés d'un macrolide (cryptate) formant une cage piégeant un atome de lanthanide.

Un exemple de lanthanide est illustré par la formule I suivante :

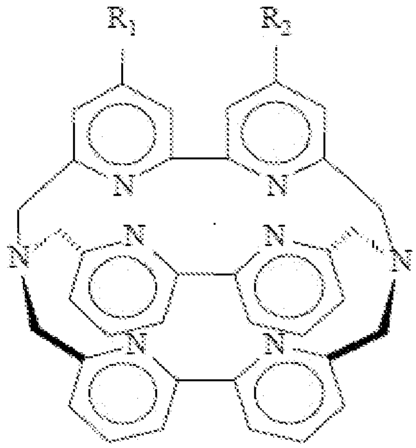

(I)

où R1 et R2 sont des groupements réactifs qui permettent de greffer le cryptate sur des biomolécules, et notamment les anticorps.

Une propriété intéressant des cryptates réside dans leur capacité à collecter l'énergie des photons d'une source lumineuse excitatrice et de la transférer sur le cation lanthanide. Ce phénomène est appelé l'effet antenne.

En outre, les cryptates de lanthanides sont particulièrement intéressant car leur longueur d'onde d'excitation se trouve dans l'ultraviolet (280 à 360 nm), tandis que leur longueur d'onde d'émission se trouve dans le vert-rouge (450 à 800 nm).

Encore un autre avantage des cryptates de lanthanides est leur capacité de fluorescence longue, c'est-à-dire qu'après excitation, ils continuent d'émettre pendant des durées variant de microsecondes à des millisecondes, alors que les fluorophores classiques émettent pendant quelques nanosecondes.

Plus particulièrement, la seconde molécule luminescente est avantageusement d2, DY647, le Cy5, l'Alexa647, ou tout autre fluorophore émettant dans le rouge lorsque la première molécule luminescente est le cryptate d'europium. Du fait de son spectre d'émission plus étendu, le cryptate de terbium peut être couplé avec une seconde molécule luminescente absorbant dans le rouge (d2, DY647, le Cy5, l'Alexa647), mais également dans le vert (la fluorescéine et les protéines fluorescentes GFP). Ces listes ne sont pas limitatives, et l'homme du métier peut, avec ses connaissances générales, déterminer les différentes première et deuxième molécules luminescentes à combiner pour obtenir un transfert d'énergie.

La molécule d2 est connue de l'état de la technique, et est notamment décrite dans le brevet US7091348 B2.

De manière avantageuse, les couples utilisés sont les suivants :

- SC450 couplé au cryptate d'europium, et le ou les anticorps donneurs sont couplés au d2, au DY647, au Cy5, à l'Alexa647 ou à toute autre molécule luminescente absorbant dans le rouge,
- SC450 couplé au cryptate de terbium, et le ou les anticorps donneurs sont couplés au d2, au DY647, au Cy5, à l'Alexa647 ou à toute autre molécule luminescente absorbant dans le rouge, mais aussi à la fluorescéine et aux protéines fluorescentes GFP, ou à toute autre luminescente absorbant dans le vert.

Les conditions expérimentales pour réaliser la méthode de l'invention, c'est-à-dire : la quantité d'anticorps utilisés, les marquages, l'exposition à la lumière excitatrice, les temps de détection, etc... sont à la portée de l'homme du métier car il s'agit d'expériences de routines.

Une fois la quantité de protéine mesurée, dans le cadre des protéines dont l'expression est contrôlée par la séquence Kozak mutée, ce niveau d'expression est comparé à celui de la même protéine mais dont l'expression est contrôlée par la même séquence Kozak ne présentant pas la mutation.

Si le niveau d'expression de la protéine dont la traduction est contrôlée par la séquence mutée est équivalent, c'est à dire le même plus ou moins 15% au niveau d'expression de la même protéine dont la traduction est contrôlée par la séquence de référence, dans ce cas la mutation sera considérée comme silencieuse.

Si le niveau d'expression de la protéine dont la traduction est contrôlée par la séquence mutée est supérieur à au moins 15% du niveau d'expression de la même protéine dont la traduction est contrôlée par la séquence de référence, dans ce cas la mutation sera considérée comme une mutation positive, dont l'effet est d'augmenter l'expression.

Si le niveau d'expression de la protéine dont la traduction est contrôlée par la séquence mutée est inférieur à au moins 15% du niveau d'expression de la même protéine dont la traduction est contrôlée par la séquence de référence, dans ce cas la mutation sera considérée comme une mutation négative, dont l'effet est de diminuer l'expression.

Dans le cadre de la méthode de l'invention, il est possible qu'une séquence Kozak étudiée présente plus d'une différence par rapport à la séquence de référence, c'est à dire présente plus d'une mutation. Dans ce cas, il sera possible de déterminer l'effet conjoint de l'ensemble de ces mutations, mais aussi de déterminer l'effet individuel de ces mutations. Pour mesurer l'effet individuel, il sera alors possible de reconstituer artificiellement chacune des mutations, par exemple par mutagenèse dirigée en utilisant des oligonucléotides portant la mutation à insérer dans la séquence de référence. Ces techniques de mutagenèse dirigée sont bien connues de l'homme du métier.

Dans un mode de réalisation avantageux, l'invention concerne une méthode de pronostic telle que définie précédemment, dans laquelle si le niveau d'expression de la protéine est plus élevé que le niveau d'expression de référence, ledit individu aura plus de chance de développer une tumeur résistante aux chimiothérapies.

Les inventeurs ont montré que les mutations qui ont un effet positif sont associées à un mauvais pronostic de réponse aux chimiothérapies. Aussi, si dans un échantillon de patient il est identifié une mutation à effet positif, le patient devrait avoir une faible réponse à la chimiothérapie, et un traitement complémentaire sera nécessaire pour faire régresser la tumeur et éventuellement entrainer une rémission.

Dans le cadre du pronostic de résistance à la chimiothérapie, il est avantageux que la méthode soit appliquée à un échantillon tumoral issu de patient, c'est à dire à partir d'une biopsie pour les tumeurs solides, ou un échantillon de sang ou de moelle osseuse pour les leucémies et les hémopathies.

Dans un mode de réalisation avantageux, l'invention concerne une méthode de pronostic telle que définie précédemment, dans laquelle si le niveau d'expression de la protéine est moins élevé que le niveau d'expression de référence, ledit individu aura plus de chance de développer une maladie neurodégénérative, une maladie cardiaque ou une infertilité.

Dans le cadre du pronostic de développement d'une maladie neurodégénérative, d'une maladie cardiaque ou d'une infertilité, notamment une infertilité masculine, il est avantageux que la méthode soit appliquée à un échantillon issu d'un individu correspondant à l'organe cible de la maladie. Toutefois, ces mutations étant souvent constitutionnelles, l'application de la méthode sur un échantillon sanguin est également appropriée.

Dans un autre mode de réalisation, l'invention concerne une méthode de pronostic telle que définie précédemment dans laquelle la séquence de référence de la séquence KOZAK :

- du gène CCND1 comprend la séquence suivante 5'-AGA GCC CCA GCC ATG GAA CAC CAG CTC-3' (SEQ ID NO : 1),
- du gène CCND2 comprend la séquence suivante 5'-GCC GGG CTG GCC ATG GAG CTG CTG TGC-3' (SEQ ID NO : 2),
- du gène CCND3 comprend la séquence suivante 5'-CGC TGC CCG AGT ATG GAG CTG CTG TGT-3' (SEQ ID NO : 3),
- du gène CCNE1 comprend la séquence suivante 5'- AGC CCC ATC ATG CCG A-3' (SEQ ID NO : 47), et
- du gène CCNE2 comprend la séquence suivante 5'- AAG AAG AGA ATG TCA AGA-3' (SEQ ID NO : 48),

Lorsque la séquence Kozak de référence est considérée à partir d'un ARN messager, cette dernière est la suivante :

- 5'- AGA GCC CCA GCC AUG GAA CAC CAG CUC -3' (SEQ ID NO : 5), pour la séquence Kozak de l'ARN messager codant la Cycline D1,
- 5'- GCC GGG CUG GCC AUG GAG CUG CUG UGC-3' (SEQ ID NO : 6), pour la séquence Kozak de l'ARN messager

codant la Cycline D2,

- 5'- CGC UGC CCG AGU AUG GAG CUG CUG UGU-3' (SEQ ID NO : 7), pour la séquence Kozak de l'ARN messager codant la Cycline D3,
- 5'- AGC CCC AUC AUG CCG A -3' (SEQ ID NO : 49), pour la séquence Kozak de l'ARN messager codant la Cycline E1, et
- 5'- AAG AAG AGA AUG UCAAGA -3' (SEQ ID NO : 50), pour la séquence Kozak de l'ARN messager codant la Cycline E2.

Il est effectivement avantageux de rechercher des mutations en 5' et en 3' du codon initiateur. Dans l'invention, on numérotera les nucléotides des séquences SEQ ID NO : 1, 2 et 3 de la manière suivante. Le A du codon initiateur ATG (ou AUG si l'on considère un ARN) correspond à la position zéro (0). Les nucléotides en 5' (à gauche) seront numérotés négativement à partir du A, et les nucléotides en 3' (à droite seront numérotés positivement à partir du A. Ainsi pour exemplifier cette nomenclature, dans la séquence SEQ ID NO : 1, le nucléotide G du codon GCC se trouve en position -9 par rapport au A de l'ATG, et le nucléotide T du codon CTC se trouve en position +13 par rapport au A de l'ATG.

Il apparaît évident pour l'homme du métier que les positions 0, +1 et +2, qui correspondent au codon ATG ne devront pas être mutée, sinon il n'y aura plus de traduction.

Les définitions susmentionnées s'appliquent *mutatis mutandis* aux séquences SEQ ID NO : 4, 5 et 6.

Avantageusement, l'invention concerne une méthode de pronostic telle que définie ci-dessus, dans laquelle la quantification de l'expression de la protéine codée par ledit gène dont la séquence KOZAK a été séquencée est réalisée par FRET.

Avantageusement, l'invention concerne une méthode de pronostic telle que définie ci-dessus, dans laquelle le séquençage de ladite séquence KOZAK est réalisée par PCR sur l'ARN messager codé par ledit gène.

L'invention concerne en outre une méthode de pronostic, notamment *in vitro,* de la résistance à une chimiothérapie d'une tumeur, ladite méthode comprenant une étape de détection d'une mutation dans la séquence KOZAK du gène *Ccnd1,* ladite mutation étant une substitution C->G en position -7 par rapport au codon ATG de la séquence SEQ ID NO : 1.

Comme cela est montré ci-après, les inventeurs ont identifié une mutation en position -7 par rapport à l'ATG de la séquence Kozak du gène codant la Cycline D1 qui a pour effet d'augmenter l'expression de la protéine. Aussi, l'identification, chez un patient atteint d'une tumeur, de cette substitution où la C de la séquence de référence est substitué par un G, sera de mauvais pronostic. En effet, du fait de la forte expression de la protéine Cycline D1, la tumeur sera résistante au traitement chimiothérapeutique.

L'invention concerne de plus l'utilisation d'une composition comprenant une paire d'oligonucléotides permettant le séquençage de la séquence KOZAK du gène codant la protéine Cycline D1, Cycline D2 ou Cycline D3, pour son utilisation dans le cadre du pronostic de résistance à une chimiothérapie d'une tumeur développée par un individu.

Avantageusement, les oligonucléotides suivants peuvent être utilisés pour obtenir la séquence de la séquence Kozak :

*Ccnd1* :

amorce sens (5'-GGGCAGCAGAAGCGAGAG-3') (SEQ ID NO : 38)
amorce antisens (5'- CGGTCGTTGAGGAGGTTG-3') (SEQ ID NO : 39)

*Ccnd2* :

amorce sens (5'- TAGCCAAAGGAAGGAGGTCA-3') (SEQ ID NO : 40)
amorce antisens (5'- AAGTAGGAGCACTGCGGAAG-3') (SEQ ID NO : 41)

*Ccnd3* :

amorce sens (5'-ATTCCACGGTTGCTACATCG-3') (SEQ ID NO : 42)
amorce antisens (5'- GCACGCACTGGAAGTAGGAG-3') (SEQ ID NO : 43)

*Ccne1 :*

amorce sens (5'- GGACAAGACCCTGGCCTC-3') (SEQ ID NO : 51)
amorce antisens (5'- GTCCTGTCGATTTTGGCCAT-3') (SEQ ID NO : 52)

*Ccne2 :*

amorce sens (5'- CTTTGTTCCCGGAGCTGTTC-3') (SEQ ID NO : 53)
amorce antisens (5'- TTTCCTCTTCTTGGCCTGGA-3') (SEQ ID NO : 54)

L'invention concerne également une composition comprenant au moins un ARN interférant dirigé contre le gène *Ccnd1,* codant la protéine Cycline D1, pour son utilisation dans le cadre du traitement d'une tumeur résistante à une chimiothérapie, les cellules de ladite tumeur comprenant une mutation dans la séquence KOZAK du gène *Ccnd1,* ladite mutation étant une substitution C->G en position -7 par rapport au codon ATG de la séquence SEQ ID NO : 1.
La séqeunce Kozak mutée susmentionnée est illustrée par la séquence suivante : 5'-AGA GC**G** CCA GCC ATG GAA CAC CAG CTC-3' (SEQ ID NO : 8).
Comme cela a été exposé plus haut, l'augmentation de la quantité de Cycline D1 par rapport à la quantité de référence a pour effet de rendre une tumeur résistante à la chimiothérapie. Aussi, pour traiter ladite tumeur de manière efficace au moyen de chimiothérapie il est avantageux de tenter de réduite la quantité de Cycline D1. Pour ce faire, l'approche consistant à inhiber l'expression de la protéine en utilisant un petit ARN susceptible de réaliser de l'interférence à l'ARN est particulièrement avantageuse.
Avantageusement, l'un au moins des 3 siRNA suivants peut être utilisé.

siRNA1 (5'-GCC AGG ATG ATAAGT TCC TTT-3') (SEQ ID NO : 44)
siRNA2 (5'-ATT GGAATA GCT TCT GGAAT-3') (SEQ ID NO : 45)
siRNA3 (5'-CCA CAG ATG TGAAGT TCA TTT-3') (SEQ ID NO : 46)

Avantageusement, l'invention concerne une composition susmentionnée pour son utilisation susmentionnée, en combinaison avec une chimiothérapie.
Avantageusment, la composition selon l'invention est utilisée avec l'un quelconque des composés suivants seul ou en combinaison : cisplatine, oxaliplatine, paclitaxel, irinotecan (SN38) et 5-FluoroUracil.
L'invention sera mieux comprise à la lecture des figures suivantes et de l'exemple ci-après.

**Légende des Figures**

La **figure 1** représente un western blot montrant l'expression de la Cycline D1 sauvage (1.) dans différents organes : A : foie, B : rein, C : poumons, D : cœur, E : œil, F : ovaires, G : rate, H : pancréas, I : cerveau. Les extraits sont normalisés avec l'actine (2.) et la tubuline (3.).
La **figure 2** représente un western blot montrant l'expression de la Cycline D1 étiquetée FLAG-HA (1.) dans différents organes : A: poumons, B : rein, C : foie, D : cœur, E : testicules, F : œil, G : rate, H : cerveau, I : pancréas. Les extraits sont normalisés avec l'actine (2.) et la tubuline (3.).
La **figure 3** représente un western blot montrant l'expression de la Cycline D1 étiquetée, révélée par l'anticorps anti HA (1.) dans deux organes : A: rate de souris Ctag/Ctag mâles, B : rate de souris Ntag/Ntag mâles, C : rate de souris +/+ mâles, D : rate de souris Ctag/Ctag femelles, E : rate de souris Ntag/Ntag femelles, F : foie de souris +/+ femelles, G : foie de souris Ctag/Ctag femelles, H : foie de souris Ntag/Ntag femelles, I : foie de souris Ctag/Ctag mâles, et J : foie de souris Ntag/Ntag mâles. Les extraits sont normalisés avec la tubuline (2.).
La **figure 4** représente un western blot montrant l'expression de la Cycline D1 étiquetée, révélée par l'anticorps anti HA (1.) dans l'œil de souris : A: Ctag/+ hétérozygotes pour la protéine Cycline D1 étiquetée en C-terminal, B : +/+, et C : Ntag/+ hétérozygotes pour la protéine Cycline D1 étiquetée en N-terminal. Les extraits sont normalisés avec la tubuline (2.).
. Les **figures 5A et 5 B** montrent que le signal HTRF peut être augmenté en utilisant plusieurs accepteurs.
La figure 5A représente un graphique montrant le signal HTRF obtenus à partir de cellules exprimant une Cycline D1 étiquetée en N-terminal pour les différentes combinaisons « donneur » « accepteur » suivantes : A : Donneur anti-FLAG + accepteur anti-HA ; B : donneur anti-FLAG + accepteur ab1 ; C : Donneur anti-FLAG + accepteur ab3 ; D : donneur anti-FLAG + accepteurs anti-HA et ab1 ; E : donneur anti-FLAG + accepteurs anti-HA et ab3 ; F : donneur anti-FLAG + accepteurs ab1 et ab3 ; G : donneur anti-FLAG + accepteurs anti-HA et ab1 et ab3 ; H : donneur anti-HA + accepteurs anti-FLAG ; I : donneur anti-HA + accepteurs ab1 ; J : donneur anti-HA + accepteurs ab3 ; K : donneur anti-HA + accepteurs anti-FLAG et ab1 ; L : donneur anti-HA + accepteurs anti-FLAG et ab3 ; M : donneur anti-HA + accepteurs ab1 et ab3, et N : donneur anti-HA + accepteurs anti-FLAG et ab1 et ab3.
La figure 5B représente un graphique montrant le signal HTRF obtenu à partir de cellules exprimant une Cycline D1 étiquetée en C-terminal pour les différentes combinaisons « donneur » « accepteur » suivantes : A : Donneur anti-FLAG + accepteur anti-HA ; B : donneur anti-FLAG + accepteur ab1 ; C : Donneur anti-FLAG + accepteur ab3 ; D : donneur anti-FLAG + accepteurs anti-HA et ab1 ; E : donneur anti-FLAG + accepteurs anti-HA et ab3 ; F : donneur anti-FLAG + accepteurs ab1 et ab3 ; G : donneur anti-FLAG + accepteurs anti-HA et ab1 et ab3 ; H : donneur anti-HA + accepteurs anti-FLAG ; I : donneur anti-HA + accepteurs ab1 ; J : donneur anti-HA + accepteurs ab3 ; K : donneur anti-HA + accepteurs anti-FLAG et ab1 ; L : donneur anti-HA + accepteurs anti-FLAG et ab3 ; M : donneur anti-HA + accepteurs ab1 et ab3, et N : donneur anti-HA + accepteurs anti-FLAG et ab1 et ab3.

La **figure 6** est un graphique montrant le ratio de la quantité de Cycline D1 étiquetée en C-terminal / la quantité de Cycline D1 étiquetée en N-terminal, dans différents organes : A : cerveau, B : œil, C : poumons, D : rein, E : cœur, F : testicules, G : pancréas, H : rate et I : foie.

Les **figures 7A à 7H** correspondent à des immunofluorescence de la Cycline D1 dans *Substantia nigra pars compacta* de souris adultes.

La figure 7A correspond à la superposition des fluorescences obtenues par marquage de l'ADN au DAPI, de la tyrosine hydroxylase et de l'étiquette HA, dans la *substantia nigra* de souris $Gcnd1^{Ctag/Ctag}$.

La figure 7B correspond à la superposition des fluorescences obtenues par marquage de l'ADN au DAPI, de la tyrosine hydroxylase et de l'étiquette HA, dans la *substantia nigra* de souris $Ccnd1^{+/+}$.

La figure 7C correspond au marquage de l'ADN au DAPI dans la *substancia nigra* de souris $Gcnd1^{Ctag/Ctag}$.

La figure 7D correspond au marquage de l'ADN au DAPI dans la *substancia nigra* de souris $Ccnd1^{+/+}$.

La figure 7E correspond au marquage de la tyrosine hydroxylase dans la *substantia nigra* de souris $Gcnd1^{Ctag/Ctag}$.

La figure 7F correspond au marquage de la tyrosine hydroxylase dans la *substantia nigra* de souris $Ccnd1^{+/+}$.

La figure 7G correspond au marquage de l'étiquette HA, dans la *substantia nigra* de souris $Gcnd1^{Ctag/Ctag}$.

La figure 7H correspond au marquage de l'étiquette HA, dans la *substantia nigra* de souris $Ccnd1^{+/+}$.

La **figure 8** représente un graphique montrant les signaux HTRF (unités arbitraires) obtenus à partir de la *substantia nigra* (B) et du *striatum* (A) de souris $Ccnd1^{Ntag/Ntag}$ 24 heures après une injection intrapéritonéale de sel (barres noires) ou de solution saline de méthamphétamine (barres blanches) à 5 mg/kg. Les barres d'erreur représentent l'écart type sur trois expériences indépendantes.

La **figure 9** est une courbe de survie de Kaplan-Meyer de souris femelles exprimant la Cycline D1 étiquetée en N-terminal (triangles noirs) comparée à la survie des souris sauvages (carrés noirs), après traitement à la 6-hydroxydopamine.

La **figure 10** est un graphique montrant la taille de l'infarctus du myocarde (en pourcentage de l'aire de risque) après ischémie et reperfusion dans des souris A : sauvages et B : exprimant la Cycline D1 étiquetée en N-terminal. Chaque point correspond à une souris.

La **figure 11** est un graphique montrant la fragmentation de l'ADN (ratio de la mesure d'apoptose de la région d'ischémie sur la région non-ischémique en unités arbitraires) après ischémie et reperfusion dans des souris A : sauvages et B : exprimant la Cycline D1 étiquetée en N-terminal.

Les **figures 12A à 12H** correspondent à des immunofluorescence de la Cycline D1 dans les testicules.

La figure 12A correspond à la superposition des fluorescences obtenues par marquage de l'ADN au DAPI, de TRA98 et de l'étiquette HA, dans des testicules de souris $Gcnd1^{Ctag/Ctag}$.

La figure 12B correspond à la superposition des fluorescences obtenues par marquage de l'ADN au DAPI, de TRA98 et de l'étiquette HA, dans des testicules de souris $Ccnd1^{+/+}$.

La figure 12C correspond au marquage de l'ADN au DAPI dans des testicules de souris $Gcnd1^{Ctag/Ctag}$.

La figure 12D correspond au marquage de l'ADN au DAPI dans des testicules de souris $Ccnd1^{+/+}$.

La figure 12E correspond au marquage de TRA98 dans des testicules de souris $Gcnd1^{Ctag/Ctag}$.

La figure 12F correspond au marquage de TRA98 dans des testicules de souris $Ccnd1^{+/+}$.

La figure 12G correspond au marquage de l'étiquette HA, dans des testicules de souris $Ccnd1^{Ctag/ctag}$.

La figure 12H correspond au marquage de l'étiquette HA, dans des testicules de souris $Ccnd1^{+/+}$.

La **figure 13** représente un graphique montrant les signaux HTRF (unités arbitraires) obtenus à partir de testicules de souris $Ccnd1^{Ntag/Ntag}$ 14 heures après une injection intrapéritonéale de sel (A) ou de solution saline d'acide méthoxyacétique à 150 mg/kg. Les barres d'erreur représentent l'écart type sur trois expériences indépendantes.

La **figure 14** représente un western blot montrant l'expression de la Cycline D1 étiquetée, révélée par l'anticorps anti FLAG (1.) dans des MEF transformées avec l'antigène T de souris : A: sans Cycline D1 (-/-), B : de souris exprimant la Cycline D1 étiquetée en N-terminal (Ntag/Ntag) et C : des souris exprimant la Cycline D1 étiquetée en C-terminal (Ctag/Ctag). Les extraits sont normalisés avec l'actine (2.).

La **figure 15** représente un western blot montrant l'expression de la Cycline D1 (1.) dans des MEF transformées avec l'antigène T de souris : A : sauvages (+/+), B : de souris exprimant la Cycline D1 étiquetée en N-terminal (Ntag/Ntag) et C : des souris n'exprimant pas de Cycline (-/-), traitée avec 0,5M d'éthanol au temps indiqués (en minutes). La quantité de PARP clivée est également détectée (2.) La quantité Les extraits sont normalisés avec l'actine (3.).

La **figure 16** est un graphique montrant le pourcentage de cellules apoptotiques dans les cellules MEF immortalisées avec l'antigène T issues de souris n'exprimant pas de Cycline D1 (-/- ; barres noires) ou de souris sauvages (+/+ ; barres blanches), après traitement A : avec une solution saline, B : après traitement à l'actinomycine D (30 $\mu$M),

C : après traitement au PD0332991 (100 nM), D : après traitement à l'actinomycine D (30 μM) et au PD0332991 (100 nM), E : après traitement à l'actinomycine D (30μM) et au Q-VD-Oph (20 μM) et F : après traitement au Q-VD-Oph (20 μM).

La **figure 17** représente un western blot montrant l'expression de la Cycline D1 (1.) dans des MEF transformées avec l'antigène T de souris : A, B : n'exprimant pas de Cycline endogène (-/-) et exprimant la Cycline D1 ectopique étiquetée en C-terminale sous contrôle d'une séquence Kozak mutée, C, D : n'exprimant pas de Cycline endogène (-/-), traitée avec 0,5M d'éthanol (B, D) ou non (A, C). La quantité de PARP clivée est également détectée (2.) La quantité Les extraits sont normalisés avec l'actine (3.).

La **figure 18** représente un western blot montrant l'expression de la Cycline D1 (1.) dans des MEF transformées avec l'antigène T de souris n'exprimant pas de Cycline (-/-) et exprimant: A: la Cycline D1 étiquetée en N-terminal sous contrôle d'une séquence Kozak mutée, B : la Cycline D1 étiquetée en N-terminal sous contrôle d'une séquence Kozak sauvage C : la Cycline D1 étiquetée en C-terminal sous contrôle d'une séquence Kozak mutée, et D : la Cycline D1 étiquetée en C-terminal sous contrôle d'une séquence Kozak sauvage. Toutes les cellules sont traitées avec de l'éthanol à 0,5M pendant 120 min. La quantité de PARP clivée est également détectée (2.) La quantité Les extraits sont normalisés avec l'actine (3.).

La **figure 19** est un graphique représentant les données obtenues par FACS de mort cellulaire en % en fonction de la quantité d'éthanol (0 ou 1000 mM) sur des MEF - /- rexprimant la Cycline D1 étiquetée en N-terminal sous contrôle d'une séquence Kozak mutée (barres noires) ou sauvage (barres blanches).

La **figure 20** est une représentation schématique des polymorphismes retrouvés dans la séquence Kozak *du gène Ccnd1* humain. La position des polymorphismes est indiquée par des S ou le M au-dessus de la séquence SEQ ID NO : 35.

La **figure 21** représente un western blot montrant la fragmentation de la PARP (1.) dans des MEF transformées avec l'antigène T de souris n'exprimant pas de Cycline (-/-) et exprimant: A et D: la Cycline D1 humaine étiquetée en C-terminal sous contrôle d'une séquence Kozak sauvage, B et C : la Cycline D1 humaine étiquetée en C-terminal sous contrôle d'une séquence Kozak mutée C/G en position -7. Les cellules sont traitées soit avec un siRNA non relevant (A et B) soit avec un siRNA anti HA (C et D). Toutes les cellules sont traitées avec de l'éthanol à 0,5M pendant 120 min. La quantité Les extraits sont normalisés avec l'actine (2.).

La **figure 22** est un graphique montrant l'induction de l'apoptose (en unité arbitraire) de cellules MEF -/- exprimant la Cycline D1 humaine sous contrôle d'une séquence Kozak mutée (-7C/G ; barres blanches) ou sous contrôle d'une séquence Kozak sauvage (barres noires) en fonction du temps (indiqué en axe des abscisses en heures) après traitement avec 500 mM d'éthanol.

La **figure 23** est un graphique représentant le pourcentage de cellules apoptotiques après traitement au 5-FU, aux doses indiquées en abscisses (en μM), de cellules MEF transformées par RAS-G12V/DNP53 n'exprimant pas de Cycline D1 (-/- ; barres noires), sauvages (barres blanches), exprimant la Cycline D1 étiquetée en N-terminal sous contrôle d'une séquence Kozak non-optimale (barres avec des hachures) ou exprimant la Cycline D1 étiquetée en C-terminal sous contrôle d'une séquence Kozak sauvage (barres grises).

La **figure 24** est un graphique montrant le pourcentage de cellules apoptotiques après traitement de cellules MEF transformées avec RAS-G12V/DNP53, A: non traitées, B : traitées au PD0332991 (500 nM), C : traitées au 5-FU (100 μM), D : traitées au 5-FU (100 μM) et au PD0332991 (500 nM) et E : traitées au 5-FU (100 μM) et au Q-VD-Oph (20 μM).

La **figure 25** est un western blot montrant la phosphorylation de RB en S780 (1.), la quantité totale de RB (2.) sur des cellules de la figure 24 non traitées (A) ou traitées avec du PD0332991 (B). Les lysats sont normalisés avec l'actine (3.).

La **figure 26** est un western blot montrant l'immunoprécipitation de la Cycline D1 à l'aide d'anticorps anti-HA (A à C), anti FLAG (E à G) ou avec des immunoglobulines non relevantes (D et H) à partir de cellules issues de tumeurs mammaires MMTV-ErbB2 après traitement pendant une minute avec 1% de paraformaldéhyde, exprimant la Cycline D1 étiquetée en N-terminal (A, C à E et G à H) ou la Cycline D1 normale (B et F). La caspase 3 est détectée (1.) ainsi que la Cycline D1 (2.),

La **figure 27** est un western blot détectant la Cycline D1 (1.) et le clivage de la PARP (2.) à partir de cellules issues de tumeurs mammaires MMTV-ErbB2 exprimant la Cycline D1 sauvage (A et B), exprimant la Cycline D1 étiquetée en N-terminal (C à I), après traitement (+) ou non (-) avec les drogues suivantes : 5-FU (100 μM ; a.), Q-VD-Oph (20 μM ; b.), un siRNA dirigé contre l'étiquette HA (c.) et un siRNA non relevant (d.). Les charges sont normalisées avec l'actine (3.).

La **figure 28** est un western blot montrant la quantité de Cycline D1 (1.) étiquetée (*) ou non (**) dans deux clones (haut et bas) de cellules MEF *Ccnd1*$^{-/-}$ exprimant : A et I : la protéine Cycline D1 étiquetée et la forme T286A de la Cycline D1, B et H: la forme T286A étiquetée de la Cycline D1 et la protéine Cycline D1, C et G : la forme T286A étiquetée de la Cycline D1, D et F : la protéine Cycline D1 étiquetée, E les cellules MEF *Ccnd1-/-*. Les cellules ont également été traitées avec un siRNA anti étiquette (F à I) où seule la forme étiquetée est sensible à l'inhibition. Les

protéines sont normalisées avec l'actine (2.).

La **figure 29** est un graphique montrant la taille des tumeurs (en $mm^3$) en fonction du temps (indiqué en axe des abscisses) obtenus chez des souris athymiques (nude) injectées avec des celles 3T3 transformées avec la forme T286A étiquetée de la Cycline D1, traitées avec un siRNA non relevant (courbe avec des losanges), avec un siRNA anti HA (courbe avec des carrés) ou anti FLAG (courbe avec des triangles). Les barres d'erreur correspondent à l'écart type sur 10 tumeurs.

La **figure 30** est un graphique montrant l'augmentation de la tumeur sur 5 jours après injection à des souris *nude* des cellules 3T3 exprimant la forme T286A de la Cycline D1 (A à C) ou la forme T286A étiquetée de la Cycline D1 (D à H) après traitement avec : A et D: un siRNA anti HA, B et E : un siRNA anti FLAG et un siRNA anti HA, C et F: un siRNA anti FLAG, G : un siRNA non relevant, et H sans siRNA. Les barres d'erreur correspondent à l'écart type sur 10 tumeurs.

La **figure 31** est un graphique montrant la taille des tumeurs (en $mm^3$) au cours du temps (en jours) obtenue à partir de souris *nude* injectées avec des cellules MEF transformées avec RAS-G12V/DNP53 exprimant la Cycline D1 et traitées avec un siRNA anti HA (courbe avec des losanges), exprimant la Cycline D1 étiquetée en N-terminal et traitées avec un siRNA anti HA (courbe avec des carrés), ou exprimant la Cycline D1 étiquetée en N-terminal et traitées avec un siRNA non relevant (courbe avec des triangles). Le traitement avec les siRNA est indiqué par la barre horizontale (Jours 13 à 17).

La **figure 32** est un graphique montrant la taille des tumeurs (en $mm^3$) au cours du temps (en jours) obtenue à partir de souris *nude* injectées avec des cellules MEF transformées avec RAS-G12V/DNP53 exprimant la Cycline D1 étiquetée en C-terminal et traitées avec un siRNA anti HA (courbe avec des losanges), ou exprimant la Cycline D1 étiquetée en N-terminal et traitées avec un siRNA non relevant (courbe avec des carrés). Le traitement avec les siRNA est indiqué par la barre horizontale (Jours 13 à 15).

**Les figures 33A à 33D** représentent des photos des tumeurs avant et après traitement avec un siRNA.

La figure 33A représente une tumeur obtenue après injection de cellules MEF RAS-G12V/DNP53 exprimant la Cycline D1 étiquetée en C-terminal.

La figure 33B représente une tumeur obtenue après injection de cellules MEF RAS-G12V/DNP53 exprimant la Cycline D1 étiquetée en C-terminal, 9 heures après traitement avec un siRNA anti étiquette.

La figure 33C représente le dos d'une souris où l'on observe sur le flanc gauche une tumeur obtenue après injection de cellules MEF RAS-G12V/DNP53 exprimant la Cycline D1 étiquetée en C-terminal et sur le flanc droit une tumeur obtenue après injection de cellules MEF RAS-G12V/DNP53 exprimant la Cycline D1.

La figure 33D représente le dos d'une souris où l'on observe sur le flanc gauche une tumeur obtenue après injection de cellules MEF RAS-G12V/DNP53 exprimant la Cycline D1 étiquetée en C-terminal et sur le flanc droit une tumeur obtenue après injection de cellules MEF RAS-G12V/DNP53 exprimant la Cycline D1, 5 jours après traitement avec un siRNA anti étiquette. La tumeur sur le flanc gauche a réduit, tandis que celle sur le flanc droit a grossi.

La **figure 34** représente un graphique montrant la taille des tumeurs (en $mm^3$) obtenues à partir de deux clones indépendants de cellules MEF RAS-G12V/DNP53 exprimant la Cycline D1 étiquetée en N-terminal et sous la dépendance d'une séquence Kozak non-optimale (A et B) ou exprimant la Cycline D1 non-étiquetée sous la dépendance de la Kozak sauvage (C et D). Les barres d'erreur correspondent à l'écart type sur 10 tumeurs.

La **figure 35** représente un graphique montrant l'augmentation des tumeurs obtenues à partir de cellules MEF *Ccnd1-/-* transformées avec RAS-G12V/DNP53 et exprimant la Cycline D1 étiquetée sous contrôle du promoteur sauvage (A) ou sous contrôle du promoteur muté (mutation -7 C/G ; B et C) pendant trois jours après traitement avec des siRNA non relevant (A et B) ou anti HA (C). Les barres d'erreur correspondent à l'écart type sur 10 tumeurs.

La **figure 36** est un western blot montrant l'expression de la Cycline D1 humaine (1.) exprimée dans des MEF *Ccnd1-/-* (A et D) ou des MEF *Ccnd1-/-* exprimant la Cycline D1 humaine étiquetée sous contrôle du promoteur sauvage (B et E), ou sous contrôle d'un promoteur muté (mutation -7 C/G ; C et F). Les cellules sont cultivées en présence (A à C) ou en absence (D à F) de sérum.

La **figure 37** représente un graphique montrant l'augmentation des tumeurs obtenues à partir de cellules MEF exprimant Large T et l'oncogène RAS-G12V sous contrôle d'une séquence Kozak optimale (B et C) ou sous contrôle d'une séquence Kozak non-optimale ; A) pendant trois jours après traitement avec des siRNA non relevant (A et B) ou anti HA (C). Les barres d'erreur correspondent à l'écart type sur 10 tumeurs.

## EXEMPLES

### Exemple 1

### Résumé

Les inventeurs ont découvert que la Cycline D1 exerçait une fonction dans les tissus adultes différenciés de

protection contre les stress environnementaux. Les inventeurs ont trouvé que la Cycline D1 agit directement par interaction avec la caspase 3 pour préserver l'intégrité de la Poly ADP Ribose Polymérase (PARP) et inhiber la mort cellulaire programmée. Malheureusement cette fonction participe au développement tumoral et augmente la résistance aux chimiothérapies indépendamment des fonctions CDK4/CDK6. La Cycline D1 est donc une pierre angulaire du développement tumoral, et la nécessité de sa diminution est une clé du traitement du cancer.

**Résultats**

***La Cycline D1 est exprimée dans les organes.***

Les inventeurs ont précédemment généré des souris FLAG-HA-Cycline D1, ou l'étiquette FLAG-HA est positionnée en C-terminal ou en N-terminal de la protéine. Ces souris se développent normalement et régulent la protéine étiquetée (que ce soit une étiquette C-terminale ou N-terminale) de la même manière que la protéine sauvage, puisque le promoteur a été maintenu. Dans des conditions normales d'entretien des souris, les deux types de souris se développent et grandissent sans développer de signes pathologiques apparents, contrairement aux animaux invalidé pour le gène *Ccnd1*. La Cycline D1, considérée comme dispensable à l'âge adulte, mais bien que cela soit difficile à démontrer la Cycline D1 sauvage est exprimée dans les organes adultes **(Figure 1).** Toutefois, la Cycline D1 étiquetée en C-terminal est facilement détectable dans les tissus adultes à l'aide de l'étiquette HA **(Figure 2** et **Figure 3).**

De manière surprenante, la protéine étiquetée en N-terminal est moins exprimée que la protéine sauvage ou la protéine étiquetée en C-terminal **(Figure 4 et Figure 2).**

Une mutation de la Cycline D1 étiquetée en N-terminale est retrouvée dans la séquence Kozak, ce qui a pour effet d'induire un défaut de traduction efficace de la protéine. En effet, les séquences codant les protéines étiquetées en N-terminal sont placées sous contrôle d'une séquence Kozak mutée 5'-GGCCGCGCC***AT***ATGG-3' (SEQ ID NO : 9) alors que les séquences codant les protéines étiquetées en C-terminal sont placées sous contrôle d'une séquence Kozak sauvage 5'-GGCCGCGCCATGG-3' (SEQ ID NO : 10).

Pour mesurer précisément la diminution de l'expression de la Cycline D1 étiquetée en N-terminal, par rapport à la Cycline D1 étiquetée en C-terminal, les inventeurs ont développé une méthode de détection de transfert d'énergie par résonnance de Förster en temps résolu (TR-FRET), fournissant une forte sensibilité de détection des protéines étiquetées. Le TR-FRET est basé sur le transfert d'énergie d'un anticorps donneur, activé à une certaine longueur d'onde, à un anticorps accepteur activé par la fluorescence émise après excitation par l'anticorps donneur. Dans les lysats de souris exprimant une protéine Cycline D1 étiquetée, le TR-FRET est possible entre un anticorps anti-FLAG donneur et un anticorps anti-HA accepteur, et inversement. En utilisant des lysats cellulaires ou d'organe, la quantification de la protéine Cycline D1 étiquetée est précise, même à des faibles niveaux, et peut encore être améliorée en choisissant des couples anticorps donneurs/anticorps accepteurs différents **(Figures 5A et 5B).** Ces résultats montrent que la protéine étiquetée en N-terminale est deux à trois fois moins exprimée que la protéine sauvage ou la protéine étiquetée en C-terminal dans la plupart des organes **(Figure 6).**

*La **Cycline** D1 protège du stress induit par des dommages tissulaires dans la substantia nigra adulte, dans le **cœur** adulte et dans les testicules adultes.*

La présence de Cycline D1 dans les tissus adultes pose la question de son rôle physiologique en dehors de la prolifération cellulaire. L'invalidation du gène codant la Cycline D1 est associée à une hyperplasie rétinienne et des désordres neurologiques. Les analyses génomiques et protéomiques dans les cellules de la rétine ont montré que la Cycline D1 interagissait avec plusieurs biomarqueurs impliqués dans l'étiologie de la maladie de Parkinson.

En effet, les inventeurs ont montré que la Cycline D1 est exprimée dans la *substantia nigra,* site ou la mort de neurones dopaminergiques est une caractéristique chez les patients atteints de la maladie de Parkinson. **(Figures 7A à 7H).** Les inventeurs ont testé cette région du cerveau chez les souris homozygotes *Ccnd1* étiquetée en N-terminal ou les souris sauvages *(Ccnd1*+/+) en utilisant de la 6-hydroxydopamine (6-OHDA), une neurotoxine utilisée pour tuer sélectivement les neurones dopaminergiques et noradrénergiques. La 6-OHDA pénètre dans les neurones à l'aide des transporteurs de monoamines, et met en péril l'intégrité cellulaire par stress oxydatif.

Les animaux sauvages survivent au traitement, tandis que les souris exprimant peu de Cycline D1 (exprimant la Cycline D1 étiquetée en N-terminal) meurent en quelques jours après le traitement 6-OHDA du fait de la mort des neurones dopaminergiques **(Figure 9).** Un autre modèle de neurones dopaminergiques testés avec la méthamphétamine conduit à une augmentation des lésions cérébrales chez les animaux exprimant peu de Cycline D1 alors que la quantité de Cycline D1 diminue dans la *substantia nigra* **(Figure 8).**

Ces résultats montrent le rôle de la Cycline D1 dans les désordres neurodégénératifs liés à l'âge de la *substantia nigra,* où elle participe au maintien de la longévité des neurones dopaminergiques en réponse aux stresses induisant la mort cellulaire.

Afin de déterminer si la quantité de Cycline D1 influence également la survie des cellules cardiaques, les inventeurs ont testé la susceptibilité des animaux exprimant la Cycline D1 étiquetée en N-terminal à une apoptose pathologique en utilisant un protocole provoquant un infarctus du myocarde. Des souris anesthésiées ont été soumises à une ischémie cardiaque *in vivo,* en réalisant une ligature réversible de l'artère coronaire. La taille de l'infarctus et la fragmentation spécifique de l'ADN (une signature de l'apoptose) sont systématiquement plus important dans les souris exprimant la Cycline D1 étiquetée en N-terminale par rapport aux souris sauvages **(Figures 10 et 11).** Ces résultats montrent la participation de la Cycline D1 dans la protection contre l'apoptose induite par un stress. Comme la caspase 3 joue un rôle clé dans l'apoptose induite par l'ischémie-reperfusion, il est possible que la Cycline D1 inhibe la cascade d'activité (signal de mort) induit par la caspase 3.

Les inventeurs ont alors testé un troisième organe en soumettant les animaux à de l'acide méthoxyacétique (MAA). Le MMA est présent dans de nombreux produits industriels et il a été démontré qu'il exerce un effet délétère sur les cellules germinales males exprimant la Cycline D1, en induisant la cascade d'activation induite par la caspase 3 **(Figures 12A à 12H).** Après une injection intra-péritonéale de MAA, les inventeurs ont observé dans les testicules de souris exprimant la Cycline D1 étiquetée en N-terminal une perte massive de marqueur de cellules germinale (Vasa) et une augmentation du taux de PARP clivées, signature de l'activation de la caspase 3, par rapport aux souris sauvages. La diminution de la Cycline D1 après traitement au MAA indique qu'elle participe à la prévention de l'apoptose induite par la caspase 3 **(Figure 13).**

Contrairement aux tissus précédemment testés, les inventeurs n'ont pas vu de différence de résistance au traitement au palmitate dans les ilots de Langerhans quelles que soient les souris testées. Les cellules β du pancréas se développent principalement *via* la Cycline D2, mais apparemment pas *via* la Cycline D1. La Cycline D2 pourrait être la Cycline D dominante dans ces cellules où la Cycline D1 est faiblement détectable **(Figures 1, 2 et 6).**

Les résultats *in vivo* montrent une fonction intrinsèque de la protéine Cycline D1 : la protection contre la mort cellulaire induite par les stress dans les organes qui l'expriment.

***La Cycline D1 inhibe le clivage de la PARP et l'apoptose indépendamment de l'activité kinase de CDK4.***

Afin de clarifier la fonction de protection contre l'apoptose de la Cycline D1 de manière indépendante de l'axe CDK4/RB, les inventeurs ont testé l'impact de la Cycline D1 dans des lignées cellulaires immortalisées pat l'antigène T. L'antigène T est connu pour bloquer à la fois les voies de signalisation P53 et RB. Dans ces cellules, les niveaux de Cycline D1 étiquetée en N-terminal restent plus bas comparés aux cellules contrôles **(Figure 14).** Les inventeurs n'ont pas remarqué de différences statistiques entre l'apoptose basale des cellules sans Cycline D1 et celle des cellules ayant peu de Cycline D1, ou l'apoptose FAS/FASL n'est pas efficace dans ces cellules.

Cependant, l'apoptose induite par l'éthanol ou l'actinomycine D, un inhibiteur de l'élongation de la transcription, augmente avec un faible niveau de Cycline D1, accompagné d'un fort clivage de la PARP **(Figure 15).**

Le PD0332991, un inhibiteur spécifique de CDK4, ou le Q-VD-Oph, un inhibiteur pan-caspases, montre que la Cycline D1 inhibe l'apoptose médiée par la caspase 3 indépendamment de CDK4 **(Figure 16).**

***La séquence Kozak de la Cycline D1 participe à la résistance à l'apoptose***

Les cellules fibroblastes embryonnaires murins MEF n'exprimant pas de Cycline D1 ont un fort taux de PARP clivée en réponse à l'éthanol comparé aux cellules contrôle, clivage qui est réversé par l'expression de la Cycline D1 **(Figure 17).**

Tenant compte des résultats avec les animaux exprimant une Cycline D1 étiquetée en N-terminal, les inventeurs ont constaté que la meilleure traduction est médiée avec la séquence Kozak sauvage, alors qu'une insertion d'un AT avant l'ATG initiateur (Kozak-) induit un plus fort clivage de la PARP en présence d'éthanol **(Figure 18**).

Dans les fibroblastes les niveaux de Cycline D1 diminuent au cours du temps après le stress, alors que le clivage de la PARP augmente, ce qui a pour conséquence que l'apoptose induite par l'éthanol est plus importante quand la quantité de Cycline D1 est suboptimale **(Figure 19).**

Au vu de l'importance de la séquence Kozak de souris pour prévenir de l'apoptose, les inventeurs ont regardé cette région dans chez l'homme. Ils ont trouvé un polymorphisme (-7C/G) dans le cœur de la séquence Kozak de la Cycline D1 humaine **(Figure 20).** La restauration de la Cycline D1 dans les MEF n'exprimant pas de Cycline D1 montre des niveaux de traduction de protéine sont élevés si la traduction est contrôlée par la séquence mutante humaine (-7G/C) par rapport à la traduction contrôlée par la séquence sauvage (SEQ ID NO : 1). Les MEF exprimant la Cycline D1 sous contrôle de la séquence mutée présentent une meilleure protection contre le clivage de la PARP **(Figure 21),** ce qui corrèle avec le fait que l'apoptose est plus réduite dans les cellules exprimant plus de Cycline D1 que celles exprimant moins de Cycline D1 **(Figure 22),** et confortant l'idée que la Cycline D1 possède des propriété anti apoptotiques.

***La Cycline D1 inhibe l'apoptose induite par la chimiothérapie***

Au vu de la prévalence de l'augmentation de la Cycline D1 dans les pathologies humaines, les inventeurs ont évalué l'impact de la Cycline D1 et de son invalidation dans la survie des cellules cancéreuses. Des MEF *Ccnd1-/-, Ccnd1*Ctag/Ctag, CcndlNtag/Ntag or *Ccnd1*+/+ ont été transformées avec un dominant négatif de P53 et un mutant oncogénique de RAS (RAS G12V). Les cellules Cycline D1 nulles, ou exprimant des faibles ou des hauts niveaux de Cycline D1 ont alors été traitées au 5-fluorouracyle (5-FU), un agent intercalant, ou l'actinomycine D, qui bloque la transcription. Les inventeurs ont pu montrer que la quantité de Cycline D1 corrèle avec la diminution des taux d'apoptose, montrant que la Cycline D1 inhibe l'apoptose induite par le stress également dans les cellules tumorales **(Figure 23).**

La Cycline D1 active CDK4 pour favoriser la progression tumorale via MMTV-ErbB2, mais l'inhibition pharmacologique de CDK4 ne semble pas affecter l'apoptose médiée par RAS après traitement au 5-FU **(Figures 24 et 25).** Pour évaluer l'effet de la Cycline D1 sur la survie des cellules ErbB2, les inventeurs ont généré des cellules *Ccnd1*+/+ et CcndlNtag/Ntag à partir d'un modèle de tumeur de la glande mammaire. L'inhibition de la Cycline D1 étiquetée dans les cellules ErbB2 augmente l'apoptose induite par le 5-FU. L'apoptose induite par l'éthanol est aussi augmentée par l'invalidation de la Cycline D1 étiquetée dans les lignées RAS et ErbB2, mais sans impliquer une inhibition de CDK4. Aussi, la Cycline D1 module la mort cellulaire des cellules tumorales de manière indépendante de CDK4, mais via une activation des caspases, puisque le Q-VD-Oph shunte l'apoptose dans ces expériences **(Figures 24 et 25).**

Comparé aux fibroblastes immortalisé par l'antigène T, la confluence ou le sevrage en sérum n'affecte que faiblement le niveau de Cycline D1 dans les cellules tumorales RAS ou ErbB2. Mais, de la même manière, une résistance accrue à l'apoptose induite par le stress dans les cellules MMTV-ErbB2 ou RAS/P53 exprimant fortement la Cycline D1 est observée, et semble déconnectée des signaux mitogéniques extracellulaires. Ces résultats montrent qu'en conditions délétères, la Cycline D1 inhibe l'apoptose induite par la caspase 3 des cellules tumorales en dehors de toute régulation du cycle cellulaire.

***La Cycline D1 s'associe avec la caspase 3.***

Dans les interactomes publiés sur la Cycline D1, aucun acteur de l'apoptose n'a été décrit. Comparé aux partenaires abondants, comme les CDK, la détection par spectrométrie de masse d'interactants transitoires de la Cycline D1 est difficile du fait de leur faible représentativité. Aussi, les inventeurs ont conçu une technique visant à figer les interactions, par une fixation chimique partielle, afin de révéler de nouveaux partenaires de la Cycline D1. En utilisant des cellules tumorales MMTV-ErbB2 fixées à la formaldéhyde, les inventeurs ont réalisé une capture de la Cycline D1 et analysé par spectrométrie de masse les interactants, et ont identifié la caspase 3. Les inventeurs ont vérifié l'interaction Cycline D1/Caspase 3 qui semblait indétectable en capturant la Cycline D1 étiquetée en C-terminal à partir de cellules RAS/P53 sans fixation chimique préalable, ce qui peut expliquer pourquoi la caspase 3 n'a jamais été retrouvée associé à la Cycline D1 dans les interactomes précédents **(Figure 26).**

La caspase 3 est la caspase effectrice majeure de l'apoptose. La PARP joue un rôle fondamental dans le contrôle de l'intégrité du génome en catalysant la réparation du génome et est un substrat de la caspase 3. Les niveaux de PARP clivée augmentent dans les cellules cancéreuse RAS ou ErbB2 après traitement au 5-FU, bien que limités par la Cycline D1, ce qui confirme l'effet inhibiteur de la Cycline D1 sur l'activation de la caspase 3 dans les cellules tumorales **(Figure 27).**

***L'utilisation de siRNA montre que la Cycline D1 est le talon d'Achille des tumeurs***

Au vu de l'environnement peu favorable dans lequel se développent les cancers, les inventeurs considèrent qu'inhiber l'expression de la Cycline D1 par interférence à ARN impactera la croissance tumorale.

Les inventeurs ont d'abord restauré l'expression de la Cycline D1 dans des MEF n'en exprimant pas, en forçant l'expression d'une Cycline D1 hyperstable (mutant T286A) et ont montré l'effet spécifique de siRNA dirigés contre les étiquettes (**Figure 28**). Ils ont alors transformé des cellules sauvages 3T3 avec le mutant T286A, et ont implanté les cellules dans des souris *nude.* Les siRNA anti HA ou anti FLAG montrent un effet sur la progression de la tumeur **(Figures 29 et 30).**

Ensuite, en utilisant des MEF exprimant la Cycline D1 étiquetée en C-terminal, étiquetée en N-terminal ou exprimant la Cycline D1 sauvage, et transformées par RASV12 et P53 dominant négatif, les inventeurs ont montré que les siRNA contre les étiquettes inhibent rapidement les tumeurs sauf celles exprimant la protéine sauvage **(Figures 31, Figure 32 et figures 33A à 33D).** Ces résultats montrent que la Cycline D1 participe au développement et au maintien des tumeurs à partir de ces lignées cellulaires.

Ensuite, dans un modèle de cellules dérivé de croisement MMTV-ErbB2 x CcndlNtag/Ntag ou MMTV-ErbB2x*Ccnd1*+/+, l'inhibition de la Cycline D1 étiquetée en N-terminal par des siRNA anti-étiquette induit également une régression tumorale. La Cycline D1 est donc une cible de premier choix contre le cancer.

Les inventeurs ont remarqué que la progression tumorales des tumeurs RAS-G12V/DNP53 exprimant de faibles niveaux de Cycline D1 (Cycline D1 étiquetée en N-terminal), du fait de la présence d'une séquence Kozak déficiente, progressent plus lentement comparées aux tumeurs exprimant la protéine sauvage ou la protéine étiquetée en C-terminal **(Figure 34).** Pour évaluer l'impact oncogénique de la mutation de la séquence Kozak, les inventeurs ont testé des formes humaines étiquetées de la Cycline D1 exprimées fortement ou faiblement **(Figure 35).** Ces protéines humaines sont fonctionnelles et répondent aux stimuli extracellulaires **(Figure 36).** De manière intéressante, des MEF transformées par RAS-G12V/DNP53 et n'exprimant pas de Cycline D1 ne permettent pas le développement de tumeurs, et sont éliminées du site d'injection, confirmant que la Cycline D1 est nécessaire pour la formation de la tumeur dans ce modèle. Toutefois, la réintroduction de la protéine humaine Cycline D1 permet le développement de tumeurs, de manière dose dépendante, suggérant que la quantité de Cycline D1 humaine, et de fait la mutation augmentant l'expression de la protéine, est de mauvais pronostic **(Figure 34).** Enfin, l'invalidation par siRNA de la Cycline D1 humaine montre que la Cycline D1 joue un rôle dans le maintien de la tumeur **(Figure 34).**

L'oncogène RAS est aussi considéré comme une bonne cible pour inhiber le développement tumoral. Les inventeurs montrent que des mutations de la séquence Kozak ou une invalidation par siRNA peut interférer avec le développement de la tumeur sous l'influence de RAS-G12V. Dans la même logique, les inventeurs ont constaté une progression tumorale rapide des tumeurs exprimant beaucoup de RAS (sous contrôle d'une séquence Kozak de RAS augmentant l'expression) comparé aux tumeurs exprimant une quantité plus faible de RAS (sous contrôle d'une séquence Kozak normale), et l'invalidation par siRNA dirigé contre RAS réduit la tumeur **(Figure 37).**

***Conclusion***

Les inventeurs ont décrit un rôle *in vivo* inattendu de la Cycline D1 dans les organes différenciés en réponse à l'apoptose induite par un stress. L'interaction avec la caspase 3 et l'inhibition du clivage de la PARP relient directement la Cycline D1 au contrôle de l'instabilité génomique. L'inhibition de la caspase 3, en réponse à un stress environnemental semble être bénéfique dans les tissus sains adultes, mais peut être au contraire néfaste dans les cellules tumorales en créant une résistance aux chimiothérapies. Cette nouvelle fonction de la Cycline D1 est impliquée dans la réparation de l'ADN et l'induction de l'instabilité génomique des oncogènes. Cela signifie qu'indépendamment de CDK4, les cellules utilisent la Cycline D1 pour 1-réparer les dommages de l'ADN, 2- pour inhiber l'apoptose induite par les chimiothérapies, et 3- pour contrôler l'instabilité génomique. La régulation de l'apoptose par la Cycline D1 est donc un nouvel angle thérapeutique à prendre à considération dans le cadre du traitement des tumeurs. Toutefois, de tels traitements ciblant la Cycline D1 devraient être utilisés avec précaution afin de tenir compte de la fonction normale de la Cycline D1 dans les tissus sains.

Les autres Cyclines de la famille ont des propriétés anti apoptotiques similaires. Par exemple la Cycline D2 a un effet dans les cellules du pancréas, et la Cycline D3 dans les cellules hématopoïétiques.

**Matériels et Méthodes**

***Souris***

Les souris CcndlNtag/Ntag et CcndlCtag/Ctag ont été décrites précédemment dans Bienvenu, F., et al., Nature, 2010. 463(7279): p. 374-8. Les fonds génétiques C57BL/6J et 129v ont été obtenus par au moins trois croisement des descendances entre elles. Les animaux ont été obtenus selon des procédures agréées (Institut de génomique fonctionnelle; accord A 34-513) et approuvé par le comité régional d'éthique (accord CEEA-LR-12070). Les souris ont été élevées à l'institut de Génomique fonctionnel dans des conditions de luminosité de 12 heures par jour, à température stable de 22 ± 1°C, dans des conditions d'humidité contrôlées (55 ± 10%).

***Génotypage***

Le génotypage est décrit Bienvenu, F., et al., Nature, 2010. 463(7279): p. 374-8.

***Cellules de fibroblastes embryonnaires murins (MEF)***

Les MEF *Ccnd1-/-* ont été gracieusement données par Piotr Sicinski.

Les MEF ont été cultivées dans du milieu de Dulbecco (DMEM ; 41966-029, Gibco) complété avec 10% de sérum de bœuf (Life technology) et 1000U/mL de pénicilline-streptomycine (P/S) (Gibco). Toutes les lignées cellulaires ont été incubées dans un incubateur à 37°C dans une atmosphère comprenant 5% de $CO_2$, et maintenues dans des conditions sub-confluentes. Quelles que soient les conditions, les MEF ont été immortalisées avec l'antigène T ou transformées avec une combinaison RasV12 et un dominant négatif de p53.

***Cohortes de souris.***

Les souris *Ccnd1*Ntag/Ntag and CcndlCtag/Ctag ont été fournies par P. Sicinski.

Les animaux ont été croisés entre eux en utilisant des femelles C57BL/6J ou 129Sv de Charles River. Puis des cohortes d'homozygotes C57BL/6J ont été obtenues à partir du croisement de mâles C57BL/6J *Ccnd1*Ntag/+ et de femelles C57BL/6J *Ccnd1*Ntag/+, ou à partir mâles C57BL/6J *Ccnd1*Ctag/+ et de femelles C57BL/6J *Ccnd1*Ctag/+. Des cohortes d'homozygotes 129SV ont été obtenues à partir du croisement de mâles 129SV *Ccnd1*Ntag/+ et de femelles 129SV *Ccnd1*Ntag/+, ou à partir mâles 129SV *Ccnd1*Ctag/+ et de femelles 129SV *Ccnd1*Ctag/+. Si aucun fond génétique n'est précisé il s'agit d'un mélange des deux fonds génétiques (1 :1).

***Traitement des souris.***

Avant les expériences, les souris ont été manipulées comme décrit précédemment dans Ares-Santos, S., et al., Neuropsychopharmacology. 2014, 39(5): p. 1066-80.

La méthamphétamine a été dissoute dans une solution saline à 0,9% (poids/volume) de NaCl, et injecte à raison de 10mL/Kg. Plusieurs doses de méthamphétamine (3 doses de 5 mg/kg à 3h d'intervalle) ont été injectées par voie intrapéritonéale, la première dose étant injectée le midi. Les souris témoin ont été traitées avec la solution saline seule. Les souris ont été sacrifiées 24h après traitement, pour les analyses post-mortem.

L'acide méthoxy-acétique 98% (194557-50G, Sigma-Aldrich) a été dissout dans une solution saline à 0,9% (poids/volume) de NaCl, et une simple injection intrapéritonéale (150mg/kg) a été injectée aux mâles. Les souris contrôles ont été traitées avec la solution saline seule. Les souris ont été sacrifiées 14h après traitement, pour les analyses post-mortem.

***Lésions 6-OHDA**.*

Les souris ont été anesthésiées avec un mélange de kétamine (Imalgene 500, 50 mg/mL, Merial), 0,9% de solution saline (poids/vol) et de la xylazine (Rompun 2%, 20 mg/ml, Bayer) (2:2:1, i.p., 0.1 mL/30 g) et montées sur un appareil stéréotaxique. La 6-OHDA-HCl (Sigma) a été dissoute dans 0,02% d'acide ascorbique dans une solution saline à une concentration de 3μg de 6-OHDA/μL. Chaque souris a reçu deux injections unilatérales de 6-OHDA (2 μL/injection) dans le striatum dorsal droit, selon les coordonnées suivantes (en mm) : antéro-posterieur +1, médio-latéral -2,1, dorso-ventral -3,1 and antéro-postérieur +0.3, medio-latéral -2,3, and dorso-ventral -3,1. Les animaux sont alors relâchés et ont reçu une injection quotidienne de solution de glucose (50 mg/mL, sous cut.).

***Préparation des tissus pour l'immunofluorescence.***

Les souris ont été anesthésiées avec du phénobarbital (500 mg/kg, i.p., Sanofi-Aventis, France), et perfusées par voix transcardiaque avec 4% de paraformaldéhyde (poids/volume) dans 0,1M de solution saline (PBS, ph 7,5). Les cerveaux et les testicules ont été post-fixés dans la même solution et stockés à 4°C.

***Immunofluorescence des cerveaux***

Des coupes de 30 μm ont été obtenues avec un vibratome (Leica, France) et stockées at -20°C dans une solution comprenant 30% (vol/vol) d'éthylène glycol, 30% (vol/vol) de glycérol, et 0,1 M de tampon sodium phosphate, jusqu'à ce que les immunofluorescences soient réalisées. Les coupes ont été traitées comme suit: les coupes sont rincées dans du tampon Tris salin (TBS: 0,25 M Tris et 0,5 M NaCl, pH 7,5), incubées pendant 5 min dans du TBS contenant 3% d'$H_2O_2$ et 10% de méthanol (vol/vol), et ensuite rincées trois fois pendant 10 min dans du TBS. Après 15 min d'incubation dans 0,2% (vol/vol) de Triton X-100 dans du TBS, les coupes sont rincées encore trois fois dans du TBS. Les coupes sont alors incubées pendant 1 h dans une solution de BSA 3% dans du TBS. Finalement, les coupes sont incubées toute la nuit ou pendant 48h à 4°C avec les anticorps primaires: anti-tyrosine hydroxylase de souris (TH) (1:1000, Millipore), anti-dopamine transporteur de rat (DAT) (1:1000, Millipore). Après incubation avec les anticorps primaires, les coupes sont rincées trois fois pendant 10 min dans du TBS et incubées pendant 45 min avec des anticorps de chèvre ou d'âne couplés avec Cy3- ou Cy5 (1:400, Jackson Lab). Les coupes sont alors rincées pendant 10 min deux fois dans du TBS et deux fois dans du TB (0,25 M Tris) avant d'être montées dans du 1,4-diazabicyclo-[2.2.2]-octane (DABCO, Sigma-Aldrich) ou du DPX (Sigma-Aldrich).

***Immunofluorescence des cerveaux***

Des coupes de trois micromètres ont été obtenues avec un vibratome (Leica, France) et stockées à -20°C dans

une solution contenant 30% (vol/vol) d'éthylène glycol, 30% (vol/vol) de glycérol et 0,1M de tampon phosphate jusqu'à leur utilisation pour l'immunofluorescence. Le coupes ont été traitées comme suit: **Jour 1:** Les coupes ont été rincées avec une solution saline TBS (TBS; 0,25 M Tris et 0,5 M NaCl, pH=7.5). Puis les coupes ont été incubées à 80°C dans un tampon citrate à pH 6 content 0,02% de Tween, pendant 30 min et rincées trois fois avec la solution TBS. Après incubation pendant 30 min dans un tampon TBS contant 0,3% de Triton X-100, les coupes ont été rincées trois fois dans du TBS et bloquées pendant 1h dans du TBS comprenant 3% d'albumine de sérum bovin (BSA) ou 3% de sérum d'âne. Les coupes ont été incubées dans une solution de 1% BSA, 0,15% Triton X-100 dans du TBS pendant 12 à 72 heures avec différents anticorps dirigés contre la tyrosine hydroxylase (TH) de souris (1:1000, Millipore) ou contre l'étiquette HA (1:500, 715500, Life technologies). **Jour 2** : Les coupes ont été rincées trois fois pendant 10 min dans du TBS et les anticorps secondaires d'âne anti lapin marqués à l'Alexa Fluor® 594 (1:500, A-21207 Molecular Probes) ou d'âne anti souris marqués à l'Alexa Fluor® 488 ont été incubés pendant 45 min. Les noyaux ont été marqués au 4',6'-diamidino-2-phénylindole (DAPI ; 1:5000). Les coupes ont été rincées deux fois pendant 10 min avec du TBS et deux fois avec un tampon Tris (0.25 M Tris, pH=7.5), avant d'être montées sur lame avec du FluorSave™ (345789, Merck Millipore).

Les tests d'immunofluorescence des testicules ont été décrits dans Malki, S., et al. EMBO J, 2005. 24(10): p. 1798-809.

Brièvement, les testicules ont été inclus dans de la paraffine et des coupes de 5 μm d'épaisseur ont été réalisées. Après réhydratation, l'anticorps primaire anti TRA98 (1:500 fourni pas B. Boizet) ou HA (715500, Invitrogen) a été utilisé, et les anticorps secondaires comme mentionné ci-dessus ont été utilisés. Les coupes confocales ont été analysées par microscopie (LSM780, or AxioImager Z1-Dr, Zeiss).

***Préparations chirurgicales pour l'ischémie reperfusion du myocarde***

Les ischémies et reperfusion du myocarde ont été réalisées sur des souris CcndINtag/Ntag et en contrôle les souris *Ccnd1*+/+. Les souris ont été anesthésiées par injection intramusculaire d'un mélange de kétamine (50 mg/kg; Imalgène® 500; Merial, France), de xylazine (10 mg/kg; Rompun® 2%; Bayer, France) et de chlorpromazine (1.25 mg/kg; Largactil® 5 mg/ml; Sanofi-aventis, France) et ventilées par incubation trachéale avec un respirateur pour rongeurs de Harvard (volume courant 7,2 μl/g de masse corporelle; 200 respiration par min). La température a été maintenue entre 36,8 et 37°C. Après une seconde injection de kétamine (50 mg/kg) et xylazine (10 mg/kg), la poitrine est ouverte par une trachéotomie réalisée à gauche et l'artère coronaire gauche est ligaturée par pause d'élément prothétique d'occlusion. Après 5 min de stabilisation thermique, tous les animaux sont laissés pendant 30 min en ischémie. La reperfusion est obtenue en retirant le dispositif occlusif de l'artère. A la fin de la reperfusion, l'artère coronaire est religaturée et 0,10 mL de phtalocyanine bleue est injectée dans la cavité ventriculaire pour perfuser les portions du myocarde non ischémique. Les cœurs sont prélevés, disséqués (l'atrium et le ventricule droit sont retirés) et étudiés pour le mesure de l'infarctus et de la fragmentation de l'ADN.

***Mesure de la taille de l'infarctus***

Les cœurs sont disséqués et les ventricules gauches sont coupés transversalement en coupes de 1mm et incubés dans une solution de 1% de TTC (2,3,5-triphenyltetrazolium chloride; Sigma Aldrich) pendant 15 min à 37°C. Après fixation dans un tampon phosphate salin comprenant 4% de paraformaldéhyde (4% PFA-PBS), les coupes sont pesées et chaque face est photographiée avec une caméra Olympus. L'air de risque ischémique (aire non traitée en bleu) et l'aire de l'infarctus (non marquée par le TTC) sont mesurées par planimétrie en utilisant Image J (Scion corp., Frederick, MD). La taille de l'infarctus est exprimée en pourcentage de l'aire de risque ischémique.

***Mesure de la fragmentation de l'ADN après infarctus du myocarde***

La fragmentation spécifique de l'ADN est quantifiée dans des échantillons transmutals des aires ischémiques ou non avec le kit enzyme-linked immunosorbent assay (Roche Diagnostics) utilisé pour mesurer la quantité d'ADN lié au nucléosome. Des échantillons transmurals de 30 mg de l'aire non ischémique du ventricule gauche et des aires ischémiques sont prélevés des souris. Les tissus sont dilacérés dans 400 μL de tampon fourni dans le kit. Les homogénats obtenus sont centrifugés à 13000g pendant 10 min. Le surnageant est utilisé comme source d'antigènes pour un test ELISA. Le tampon d'incubation et un complexe ADN/Histones sont utilisés en contrôles négatifs. Deux valeurs de double absorbance (405 nm / 490 nm) ont été obtenues pour réaliser une moyenne ; et le bruit de fond a été soustrait de chacune de ces moyennes. Pour normaliser la fragmentation d'ADN, les nucléosomes solubles issus de régions ischémiques et non ischémiques ont été testés.

***Test de renutrition.***

Les niveaux de glucose sanguin ont été mesurés à l'aide d'un glucomètre OneTouch Ultra (LifeScan, Issy les Moulineaux, France) après une nuit de sevrage et trois heures de renutrition.

***Activité caspase dans les ilots pancréatiques***

L'activité caspase a été mesurée dans des ilots de souris en utilisant le kit Caspase-Glo 3/7 assay (Promega) selon les recommandations du fabricant. Brièvement, 48 heures après le traitement avec 0,5mM de palmitate, des groupes de 6 ilots sont transférés dans des plaques 96 puits dans 100 μL de milieu de culture. Les ilots sont alors lysés avec 100 μL de réactif Caspase-Glo 3/7 et incubés à température ambiante pendant 30 minutes. L'activité luciférase est mesurée avec un lecteur de microplaque Infinite M200 Tecan.

***Injection in vivo des siRNA et mesure de la taille des tumeurs.***

Les siRNA ont été dissouts dans de l'eau sans nucléases et stockées à -20°C avant utilisation. Une solution d'Aonys® (MedesisPharma) a été préparée extemporanément. Selon les instructions du fabricant, le mélange Aonys®/siRNA a été obtenu par mélange à l'aide d'un vortex. Le mélange est laissé à température ambiante avant utilisation.
Le mélange (1mg/mL) Aonys®/siRNA a été injecté par voie rectale à l'aide d'une micropipette à un volume constant de 20 μL par dose.
Le traitement des tumeurs par les siRNA a été réalisé deux fois par jour (matin et après-midi) tous les jours. La taille des tumeurs a été mesurée en utilisant la formule suivante : $L \times W^2/2$ ou L est la longueur et W la largeur de la masse tumorale.

***Xénogreffes***

Cellules 3T3 transformées par: $2.10^6$ cellules par implantation.
MEF transformées par RAS-G12V/DNP53: $0,5.10^6$ cellules par implantation.
Les MEF immortalisées par l'antigène T: $0,5.10^6$ cellules par implantation.
Chaque type cellulaire est repris dans 150 μL de RPMI 1640 et injecté dans le flanc de souris athymiques de 6 semaines en sous-cutané.

***Cellules***

Les MEF sont cultivées dans du DMEM comprenant 10% de sérum de bœuf fœtal, et 1000U de Pénicilline-Streptomycine. Les cellules primaires dérivées des tumeurs sont maintenues dans du DMEM comprenant 5% de sérum et 1000U de Pénicilline-Streptomycine. Toutes les cellules sont cultivées à 37°C en présence de 5% de $CO_2$. Lorsque cela est précisé, le sérum est retiré pendant 36 heures.

***Cultures primaires de tumeurs mammaires MMTV-ErbB2***

Les tumeurs sont prélevées sur les animaux et lavées au PBS 1x et découpées. Les morceaux de tumeurs sont repris dans un mélange de trypsine (1,25mg/mL), de collagénase de type I (1000 unités/mL) et de Pénicilline-Streptomycine (1000 unités/mL) dans du milieu DMEM/F-12 à 37°C pendant 20 min sous agitation. Puis les tissus dissouts sont resuspendus un cocktail d'inhibiteur de trypsine (0,5 mg/mL) et de DNAse I (370 unités/ml).
Les cellules épithéliales et fibroblastiques sont séparées par sédimentation à 1000g pendant 10 min et reprises dans du milieu, puis ensemencées dans du DMEM contenant 5% de sérum et les antibiotiques et laissées 24 à 48h en culture. Les cellules sont maintenues à un stade sub-confluentes et l'expression du transgène MMTV-ErbB2 est mesuré par RT-PCR.

***ARN.***

Les ARN sont extraits avec du Trizol (Invitrogen) selon les instructions du fabricant. 1μg d'ARN total est utilisé pour la reverse transcription en présence de 200 U de reverse transcriptase de M-MLV (Invitrogen) en présence de 2,5μM d'hexamères aléatoires, 0,5 mM de dNTP, 10mM de DTT et 40U d'inhibiteur de RNAse (Invitrogen).
Les gènes de référence utilisés sont les suivants :

| Gène | SéqRef | Forward | Séquence sens |
|---|---|---|---|
| Aldo3 | NM_009657 | mAldo3-F | CGGCACTGGCCATATTGG (SEQ ID NO : 13) |
| B2μg | NM_009735 | B2m-F | TATGCTATCCAGAAAACCCCTCAA (SEQ ID NO : 14) |
| GAPDH | NM_008084 | Gapdh-F | GGAGCGAGACCCCACTAACA (SEQ ID NO: 15) |
| Gus | NM_010368 | Gus2-F | GATTCAGATATCCGAGGGAAAGG(SEQ ID NO : 16) |
| Hprt | NM_013556 | Hprt2-F | GCAGTACAGCCCCAAAATGG (SEQ ID NO: 17) |
| Mrpl32 | NM_029271 | Mrpl32-F | AGGTGCTGGGAGCTGCTACA (SEQ ID NO: 18) |
| Tbp | NM_013684 | Tbp2a-F | ATCGAGTCCGGTAGCCGGTG (SEQ ID NO: 19) |
| Tubuline | NM_023716 | Tubb2b-F | CTTAGTGAACTTCTGTTGTCCTCCAGCA (SEQ ID NO : 20) |
| mCycline D1 | NM_007631 | *Ccnd1*-F | AGGAGCAGAAGTGCGAAGAG (SEQ ID NO: 21) |
| hCycline D1 | NM_053056 | h*Ccnd1*-F | GGCGGAGGAGAACAAACAGA (SEQ ID NO: 22) |

***Western blot et immunoprécipitations***

Les anticorps utilisés sont les suivants: anti HA (HA.11 Clone 16B12, Eurogentec ou Anti-HA EPITOPE TAG - 600-401-384,Tebu-bio or Hemagglutinin (HA) Rabbit Polyclonal Antibody, Life technologie), anti Cycline D1 (sc-450, Santa cruz or MS-210-PABX (AB1), Fisher scientific or RB-010-PABX (AB3), Fisher scientific), anti RB total (sc-74562, Santa cruz), anti Phospho-RB (Ser780) (9307, Cell signaling), anti Actine (ab6276, Abcam), anti Tubuline (T9026, Sigma-Aldrich), anti Ras (BD610002, BD Biosciences), anti FLAG (F7425, Sigma-Aldrich), anti PARP clivée (ab32064, Abcam), anti VASA (ab13840, Abcam), anti Thyrosine Hydroxylase (MAB318, Merck Millipore). Les anticorps secondaires utilisés sont couplés à la peroxydase (Cell signaling), e la détection des complexes est réalisée à l'aide du kit enhanced chemiluminescence detection (Millipore).

***Constructions.***

Toutes les constructions Cycline D1 ou RAS sont insérées entre les sites BamH1-EcoR1 des vecteurs rétroviraux pBABE-Puro founi par P. Sicinski ou MSCV fourni par O. Ayrault. Le plasmide codant l'antigène T a été fourni par L. Fajas, le plasmide codant RasV12/dominant négatif de p53 a été fourni par L. LeCam.

Toutes les constructions rétrovirales ont été manipulées selon les mesures de sécurité approuvées par l'institut de Génomique fonctionnelle.

L'ADN complémentaire (cDNA) Cycline D1 humain a été obtenu par RT-PCR à partir de fibroblaste de peau transmis par Jean-Marc Lemaitre.

***Mutagenèse.***

Toutes les mutagenèses ont été réalisées à l'aide du kit GeneArt® Site-Directed Mutagenesis System (Life-Technologies) selon les recommandations du fabricant. Les oligonucléotides utilisés sont les suivants :

| **Oligonucléotides sens** | **Séquence** |
|---|---|
| Mutag-mCcndl T286A- | GGTCTGGCCTGCGCGCCCACCGACGTG (SEQ ID NO : 23) |
| Flag-hKoz-SNP7-hCD1- | GACGATGACAAGGGAAGAGCGCCAGCCATGGAAC ACCAGCT (SEQ ID NO : 25) |
| hKOZ-SNP-hCD1- | gGGATCCggaagagcGccagccATGGAACAC (SEQ ID NO : 27) |
| mKoz-m*Cend1*- | CAGTGTGGTGGTACGGCGGCCGCGCCatgGAACAC CAGCTCCT (SEQ ID NO : 29) |
| mKoz-Flag-HA-m *Ccnd1*- | CCAGTGTGGTGGTACGGCGGCCGCGCCatggactaca aggacga (SEQ ID NO : 31) |

(suite)

| Oligonucléotides sens | Séquence |
|---|---|
| mKoz-AT-Flag-HA-m *Ccnd1*- | CCAGTGTGGTGGTACGGCGGCCGCGCCATatggacta caaggacga (SEQ ID NO : 33) |
| **Oligonucléotide antisens** | **Séquence** |
| Mutag-mCcndl T286A- | CACGTCGGTGGGCGCGCAGGCCAGACC (SEQ ID NO : 24) |
| Flag-hKoz-SNP7-hCD1- | AGCTGGTGTTCCATGGCTGGCGCTCTTCCCTTGTC ATCGTC (SEQ ID NO : 26) |
| hKOZ-SNP-HCD1- | GTGTTCCATggctggCgctcttccGGATCCc (SEQ ID NO : 28) |
| mKoz-m*Ccnd1*- | AGGAGCTGGTGTTCcatGGCGCGGCCGCCGTACCA CCACACTG (SEQ ID NO : 30) |
| mKoz-Flag-HA-m *Ccnd1*- | tcgtccttgtagtccatGGCGCGGCCGCCGTACCACCACAC TGG (SEQ ID NO : 32) |
| mKoz-AT-Flag-HA-m *Ccnd1*- | tcgtccttgtagtccatATGGCGCGGCCGCCGTACCACCAC ACTGG (SEQ ID NO : 34) |

***Génération de lignées cellulaires stables.***

Les cellules obtenues par infection rétrovirale ont été décrites dans Bienvenu et al., Nature, 2010. 463(7279): p. 374-8. Brièvement, le jour avant l'infection, les cellules Plat-E sont ensemencées dans des boites de 10 cm à 50% de confluence dans du DMEM (Gibco) additionné de sérum fœtal de bovin.

Les rétrovirus écotropes murins ont été produits par transfection au jetPEI de cellules Plat-E avec 3μg de vecteurs pBabe-puro ou MSCV-puro, ou avec un vecteur ne portant pas de gène de résistance. Quarante-huit heures après la transfection, le surnagent viral a été prélevé, filtré (0,45 μm) et additionné de polybrene (H9268, Sigma) et utilisé pour infecter les cellules receveuses en prolifération. Soixante-douze heures après l'infection, le milieu de cellules receveuses a été remplacé et les cellules sont laissées en culture pendant plusieurs jours en présence de 2μg/mL de puromycine ou 150μg/mL d'hygromycine, jusqu'à la mort des cellules contrôle (qui n'expriment pas de gène de résistance).

***Transfection des siRNA.***

Les siRNA ont été administrés aux cellules avec de la Lipofectamine® RNAiMAX Transfection Reagent (Life Technologies) selon les instructions du fabricant. Les cellules à transfecter ont été ensemencées à 9 h le matin, et transfectées à 6 h du soir le même jour. Le jour d'après à 9 h du matin, les cellules ont été récoltées pour les analyses biochimiques.

***TR-FRET.***

Les organes de souris ont été lavés avec du PBS 1x à 37°C et lysés dans du tampon HTRF (Tris 10mM, EDTA 1mM, 0.05% NP-40) en utilisant un homogéniseur de cellules. Après centrifugation à 16 000 g pendant 5 min, les échantillons ont été normalisés en ajustant à la quantité totale d'ADN (nanodrop, Thermo Scientific) à 500 ng/mL.

Grâce à une quantification selon Bradford, le contenu total de protéines a été vérifié pour réaliser une équivalence entre des organes similaires ou les échantillons à tester (par exemple reins *Ccnd1*+/+ et reins *Ccnd1*+/-). Des échantillons délétés pour les deux allèles de la Cycline D1 ont été testés comme contrôles négatifs et évaluation du bruit de fond (contrôle 1). De plus, des échantillons incubés avec l'anticorps donneur seul ont été testés en parallèle (contrôle 2). La comparaison des deux contrôles pour chaque mesure donne des résultats identiques de bruit de fond.

La détection de la Cycline D1 par HTRF (étiquetée ou non) a été réalisée avec des anticorps donneurs et accepteurs selon les instructions du fabricant (Cisbio - 0,4 nM pour le donneur sauf pour l'anticorps SC450 (0,2 nM) et 6 nM en tant qu'accepteur) dans la gamme linéaire de signal HTRF (dans la fenêtre de linéarité des anticorps), afin d'éviter une saturation du signal (effet crochet) et un trop faible signal proche du bruit de fond. Les anticorps donneurs sont marqués au cryptate de terbium (Tb), et les anticorps accepteurs sont marqués avec le fluorophore XL665 ou le d2.

Les anticorps utilisés sont les suivants :

Anticorps donneurs :

- HA-Tb, 610HATAB, Cisbio
- Flag-Tb, 61FG2TLB, Cisbio
- HA-XL, 610HAXLB, Cisbio
- AB3-Tb 64CUSTAYE, Cisbio (marquage à façon de l'anticorps RB-010-PABX (AB3), Fisher scientific)
- AB1-Tb 64CUSTAYE, Cisbio (marquage à façon de l'anticorps MS-210-PABX (AB1), Fisher scientific)
- SC-450-Tb 64CUSTAZE, Cisbio (marquage à façon de l'anticorps SC-450, Santa Cruz)

Anticorps accepteurs :

- Flag-XL, 61FG2XLB, Cisbio
- AB3-d2 64CUSDAZE, Cisbio (marquage à façon de l'anticorps RB-010-PABX (AB3), Fisher scientific)
- AB1-d2 64CUSDAZE, Cisbio (marquage à façon de l'anticorps MS-210-PABX (AB1), Fisher scientific)
- SC-450-d2 64CUSDAZE, Cisbio (marquage à façon de l'anticorps SC-450, Santa Cruz).

Pour les mesures HTRF, le mélange des anticorps est ajusté à un volume de 5 μl dans du PBS 1x et ces 5 μL sont ajoutés à 5 μL d'échantillon à tester, dans une plaque noire 384 puits Greiner. Après agitation, et centrifugation, les échantillons sont incubés à 4°C toute la nuit à l'abri de la lumière (ou 1h à température ambiante (de 19 à 25 °C)).

Le lendemain matin, l'expérience est réalisée à l'aide d'un lecteur de microplaques PHERAstar FS (BMG Labtech) : après excitation avec un laser 337 nm (40 flashs par puits), les émissions de fluorescence sont mesurées à la fois à 620 nm (émission du cryptate de terbium) et a 665 nm (émission de XL665 et de d2). Les fluorescences émises sont collectées pendant 400 μs (micro secondes) après un temps de latente de 60 μs suite aux flashs, afin d'enlever le bruit de fond provenant du milieu, du signal mesuré.

L'intensité de TR-FRET est calculée comme suit

$$TR\text{-}FRET = \{(\text{ratio } 665/620)\text{échantillon}\}x104 - \{(\text{ratio } 665/620)\text{bruit}\}x104$$

Le bruit de fond correspond aux cellules marquées avec l'anticorps donneur seul, ou un échantillon n'exprimant pas la protéine cible. Pour chaque mesure, la moyenne de plusieurs expériences a été utilisée. Les données des figures correspondent à la moyenne de plusieurs expériences indépendantes +/- l'écart type, sauf indications contraires.

***Mesure de la mort cellulaire.***

La mort cellulaire est induite par une exposition à l'actinomycine D A9415, Sigma) pendant 6 h, le 5-Fluoro uracyl (Fluorouracil Winthrop, Sanofi-Aventis) pendant 24 h ou l'éthanol (20821.330, VWR) pendant 6h et inhibée par une exposition au Q-VD-Oph (SML0063, Sigma).

Les cellules sont lavées deux fois au PBS 1x, décollées à la trypsine et reprises dans un tampon de marquage (10mM d'Hepes pH=7.4, NaCl 150mM, KCl 5mM, $MgCl_2$ 1mM, $CaCl_2$ 1,8mM). Puis les cellules ont marquées à l'annexine V-isothiocyanate de fluorescéine (BD Biosciences) selon les instructions du fabricant et 0,5ug/mL d'iodure de propidium (PI ; Sigma-Aldrich P-4170). Après 15 min d'incubation dans le noir, les cellules sont analysées par cytométrie de flux. Les cellules vivantes sont Annexine V et PI doubles négatives et les cellules apoptotiques sont positives pour l'annexine V et négatives pour le PI.

***Détection de la mort cellulaire par immunologie.***

La quantification de la mort cellulaire dans les MEF est réalisée en utilisant le kit Cell Death Detection ELISAPLUS (11920685001, Roche) selon les instructions du fabricant. Les valeurs obtenues en unités arbitraires sont calculées selon la formule : unité arbitraire= Absorbance (405nm) de l'échantillon (marquage/cellules mortes)/Absorbance (405nm) du contrôle négatif correspondant. Les données correspondent à la moyenne de trois expériences indépendantes ± l'écart type. Les données statistiques sont réalisées par un test t de Student. Les valeurs <0,05 sont considérées comme statistiquement significatives.

***Analyses statistiques.***

Les moyennes de deux groupes ont été comparées en utilisant un test t de Student bilatéral.

***Peptides Caspase-3 identifiés par spectrométrie de masse.***

SGTDVDAANLR (SEQ ID NO : 11) (position: 65-75), deux spectres MS/MS
SVDSGIYLDSSYK (SEQ ID NO : 12) (position: 26-38), un spectre MS/MS

**Exemple 2 : Protocole expérimental pour tester l'impact d'une mutation de la séquence Kozak sur l'expression d'un gène**

**1- La séquence Kozak d'intérêt est fusionnée au gène rapporteur Ntag-CycD1 par clonage:**

- Des oligonucléotides sens et antisens composés

  a-de la séquence cohésive au site de restriction de l'enzyme BamH1, suivie de
  b- un site de restriction avantageux pour le criblage décrit ci-dessous, comme par exemple EcoR1, suivi de c- la séquence Kozak d'intérêt (-9 à +3 autour du codon ATG d'initiation de la traduction) dans sa forme sauvage ou dans sa forme mutée, suivie de
  c- la séquence cohésive au site de restriction de l'enzyme Xho1, sont conçus puis assemblés par appariement de base après chauffage à 98°C pendant 3 minutes suivi d'un refroidissement lent jusqu'à 4°C.

- Le vecteur rétroviral MSCV-Ntag-CycD1, exprimant

  a-un gène de sélection eucaryote, comme un gène de rapporteur fluorescent pour tri par cytométrie de flux, ou un gène de résistance eucaryote par exemple à la puromycine et
  b-le gène rapporteur Cycline D1 fusionné aux séquences peptidiques FLAG et HA en N-terminal de la protéine, est digéré par les enzymes BamH1 et Xho1 afin de l'ouvrir pour insertion de l'oligonucléotide décrit ci-dessus, tel que la séquence Kozak permettra l'initiation de la traduction du gène rapporteur en phase avec le codon ATG d'initiation de la traduction.

- Une réaction de ligation permettant l'insertion de l'oligonucléotide dans le vecteur MSCV-Ntag-CycD1 par reconstitution des sites BamH1 et Xho1, est suivie d'une étape de transformation de bactéries thermo-compétentes pour criblages des clones bactériens ayant inséré l'oligo dans le vecteur par acquisition de la résistance à l'antibiotique ampicilline dont le gène est présent dans le vecteur original MSCV-Ntag-CycD1
- Après pousse puis amplification de clones bactériens résistants à l'ampicilline, une extraction de plasmide est réalisée pour chacun de ces clones, par exemple au moyen du kit Qiagen (QIAprep Spin Miniprep Kit, Qiagen) et le produit de purification est digéré par l'enzyme EcoR1 (ou EcoR1 plus l'enzyme reconnaissant le nouveau site de restriction avantageux inclus dans l'oligonucléotide) afin d'identifier une nouvelle bande d'ADN correspondant à la bonne intégration de l'oligo dans le vecteur.
- Le vecteur d'intérêt ainsi créé est alors amplifié puis purifié pour transfection en cellules Phoenix permettant la génération de virus vecteurs de la construction souhaitée
- Les virus ainsi produits sont utilisés pour générer des lignées stables résistantes à la puromycine qui permettront la mesure de l'expression du gène rapporteur Ntag-CycD1 en situation de séquence Kozak sauvage par rapport à la séquence mutée à analyser.
- Une étape de quantification par qPCR de l'ARN messager du gène rapporteur Ntag-CycD1 permet de s'assurer d'un niveau équivalent de transcription entre les deux lignées à comparer
- Les lignées sélectionnées à la puromycine ainsi produites sont ensemencées à confluence équivalente pour semi-quantification de l'expression du rapporteur Ntag-CycD1 au moyen d'un immunoblot, de Tandem-HTRF ou tout autre méthode de quantification protéique par anticorps spécifique de Ntag-CycD1, après lyse des cellules puis extraction protéique.
- Un contrôle de charge permet de s'assurer de la bonne comparaison de quantité totale de protéines équivalente entre les deux échantillons (par exemple une protéine d'un gène de ménage comme GAPDH, Tubuline ou Actine)
- Des niveaux d'expression différents du gène rapporteur témoigne de l'impact de la mutation de la séquence Kozak sur le niveau de traduction du gène en aval à quantité d'ARN messager équivalent attestée par la mesure de qPCR faite ci-dessus

**2- La séquence Kozak d'intérêt est fusionnée au gène rapporteur Ntag-CycD1 par mutagénèse dirigée :**

- Une réaction de mutagénèse dirigée est réalisée sur la séquence Kozak (-9 à +3 autour du codon ATG d'initiation de la traduction) du vecteur MSCV-Nde1-Ntag-CycD1 permettant l'expression du rapporteur Ntag-CycD1, au moyen d'un kit de mutagénèse comme le kit GeneArt® Site-Directed Mutagenesis System de chez Thermo Fisher (Numéro de catalogue: A13282), et grâce à l'utilisation d'oligonucléotides comprenant la séquence Kozak d'intérêt à tester encadrée de 15 nucléotides en amont correspondant aux 15 nucléotides en amont de la séquence Kozak d'initiation de la traduction du rapporteur Ntag-CycD1 et de 15 nucléotides en aval correspondant aux 15 nucléotides en aval ce cette même séquence d'initiation de la traduction de Ntag-CycD1. Le nombre de 15 nucléotides est à titre indicatif et peu varier de quelques nucléotides.
- Cette étape de mutagénèse est suivie d'une étape de transformation de bactéries thermo-compétentes pour criblages des clones bactériens ayant acquis la résistance à l'antibiotique ampicilline dont le gène est présent dans le vecteur original MSCV-Ntag-CycD1
- Après pousse puis amplification de clones bactériens résistants à l'ampicilline, une extraction de plasmide est réalisée pour chacun de ces clones, par exemple au moyen du kit Qiagen (QIAprep Spin Miniprep Kit, Qiagen) et le produit de purification est digéré par l'enzyme EcoR1 et Nde1 afin d'identifier les clones ayant perdu le site Nde1 suite à la mutagénèse de la séquence Kozak du vecteur MSCV-Ntag-CycD1. Une confirmation de cette mutagénèse est effectuée ensuite par séquençage de clones positifs pour s'assurer de l'absence de mutations intempestives dans la séuquence codant pour le gène rapporteur Ntag-CycD1.
- Le vecteur d'intérêt ainsi créé est alors amplifié puis purifié pour transfection en cellules Phoenix permettant la génération de virus vecteurs des constructions souhaitées.
- Les virus ainsi produits sont utilisés pour générer des lignées stables résistantes à la puromycine qui permettront la mesure de l'expression du gène rapporteur Ntag-CycD1 en situation de séquence Kozak sauvage par rapport à la séquence mutée à analyser.
- Une étape de quantification par qPCR de l'ARN messager du gène rapporteur Ntag-CycD1 permet de s'assurer d'un niveau équivalent de transcription entre les deux lignées à comparer
- Les lignées sélectionnées à la puromycine ainsi produites sont ensemencées à confluence équivalente pour semi-quantification de l'expression du rapporteur Ntag-CycD1 au moyen d'un immunoblot, de Tandem-HTRF ou toute autre méthode de quantification protéique par anticorps spécifique de Ntag-CycD1, après lyse des cellules puis extraction protéique
- Un contrôle de charge permet de s'assurer de la bonne comparaison de quantité totale de protéines équivalente entre les deux échantillons (par exemple une protéine d'un gène de ménage comme GAPDH, Tubuline ou Actine)
- Des niveaux d'expression différents du gène rapporteur témoignent de l'impact de la mutation de la séquence Kozak sur le niveau de traduction du gène en aval à quantité d'ARN messager équivalent attestée par la mesure de qPCR faite ci-dessus

**3-La séquence Kozak d'intérêt est amplifiée par PCR avec l'ADN complémentaire du gène en aval qu'elle contrôle :**

- L'ADN complémentaire, de cellules exprimant le gène pour lequel une mutation de la séquence Kozak est à analyser, est obtenu par extraction d'ARN totaux, par exemple au moyen du kit Qiagen (RNeasy Mini Kit, Qiagen), suivi d'une réaction de Reverse Transcription classique
- Une réaction de PCR est réalisée avec cet ADN complémentaire comme matrice et avec un couple d'oligonucléotide conçu tel qu'il permettront l'amplification du gène d'intérêt précédé a-de quelques nucléotides quelconques, suivis de b- le site de restriction BamH1, suivie de c- un site de restriction avantageux pour le criblage décrit ci-dessous, comme par exemple Nde1, suivi de d- la séquence Kozak d'intérêt (-9 à +3 autour du codon ATG d'initiation de la traduction) dans sa forme sauvage ou dans sa forme mutée ; et suivi du site de restriction pour l'enzyme EcoR1 plus quelques nucléotides quelconques
- Ce produit de PCR est ensuite digéré par les enzymes BamH1 et EcoR1 puis purifié afin de permettre son insertion dans le vecteur MSCV qui suit. Il est entendu que si les sites de restrictions BamH1 et EcoR1 sont présents dans l'ADN complémentaire du gène d'intérêt, d'autres couples d'enzymes devront être sélectionnés pour une insertion dans un vecteur rétroviral adapté à ces sites.
- Le vecteur MSCV-Ntag-CycD1, exprimant a-un gène de sélection eucaryote, comme un gène de rapporteur fluorescent pour tri par cytométrie de flux, ou un gène de résistance eucaryote par exemple à la puromycine et b-le

gène rapporteur Cycline D1 fusionné aux séquences peptidiques FLAG et HA en N-terminal de la protéine, est digéré par les enzymes BamH1 et EcoR1 afin de sortir le gène rapporteur Ntag-CycD1 pour insertion du produit de PCR purifié décrit ci-dessus

- Une réaction de ligation permettant l'insertion de ce produit de PCR dans le vecteur MSCV vide par reconstitution des sites BamH1 et EcoR1, est suivie d'une étape de transformation de bactéries thermo-compétentes pour criblages des clones bactériens ayant inséré l'oligo dans le vecteur par acquisition de la résistance à l'antibiotique ampicilline dont le gène est présent dans le vecteur original MSCV-Ntag-CycD1
- Après pousse puis amplification de clones bactériens résistants à l'ampicilline, une extraction de plasmide est réalisée pour chacun de ces clones, par exemple au moyen du kit Qiagen (QIAprep Spin Miniprep Kit, Qiagen) et le produit de purification est digéré par les enzymes EcoR1 et Nde1 ou tout autre enzyme reconnaissant le nouveau site de restriction avantageux inclus dans le produit de PCR afin d'identifier une nouvelle bande d'ADN correspondant à la bonne intégration du produit de PCR dans le vecteur.
- Le vecteur d'intérêt ainsi créé est alors amplifié puis purifié pour transfection en cellules Phoenix permettant la génération de virus vecteurs de la construction souhaitée
- Les virus ainsi produits sont utilisés pour générer des lignées stables résistantes à la puromycine qui permettront la mesure de l'expression du gène d'intérêt en situation de séquence Kozak sauvage par rapport à la séquence mutée à analyser.
- Une étape de quantification par qPCR de l'ARN messager du gène d'intérêt permet de s'assurer d'un niveau équivalent de transcription entre les deux lignées à comparer
- Les lignées sélectionnées à la puromycine ainsi produites sont ensemencées à confluence équivalente pour semi-quantification de l'expression du produit du gène d'intérêt au moyen d'un immunoblot, de Tandem-HTRF ou tout autre méthode de quantification protéique par anticorps spécifique de cette protéine, après lyse des cellules puis extraction protéique
- Un contrôle de charge permet de s'assurer de la bonne comparaison de quantité totale de protéines équivalente entre les deux échantillons (par exemple une protéine d'un gène de ménage comme GAPDH, Tubuline ou Actine)
- Des niveaux d'expression différents de la protéine issue du gène d'intérêt témoigne de l'impact de la mutation de la séquence Kozak sur le niveau de traduction du gène en aval à quantité d'ARN messager équivalent attestée par la mesure de qPCR faite ci-dessus

Il est entendu que le gène rapporteur Cycline D1 est utilisé à titre d'exemple et qu'il peut être remplacé par n'importe quel gène rapporteur comme le gène codant pour la Luciférase, la GFP ou tout autre marqueur dont la mesure de l'expression est avantageuse pour tester l'impact de la séquence Kozak sur la traduction.

Il est aussi entendu que ces méthodes de clonage classiques peuvent être remplacées par des méthodes plus récentes d'assemblage de séquences comme par exemple l'assemblage de Gibson ou encore la recombinaison homologue dirigée par la recombinase CRE2. On peut par exemple imaginer la mise en place de séquences Kozak à tester de façon isogénique au sein du génome d'une lignée cellulaire qui possèderait des sites de recombinaison LoxP pour la recombinase CRE qui entoureraient une séquence Kozak en amont du gène rapporteur souhaité.

**Exemple 3 : implication des cyclines dans les pathologies**

L'impact de mutations de séquences Kozak dans les gènes de Cycline D2, Cycline D3, Cycline E1 et Cycline E2 sur la biologie de la cellule est anticipé au regard de la nécessité de la Cycline D1 pour la survie cellulaire par inhibition de la Caspase 3, qui est un effecteur central de la mort cellulaire programmée dite par apoptose. Ces Cyclines sont redondantes dans leurs fonctions lors du développement et à l'âge adulte. Par exemple, le remplacement du gène de Cycline D1 par le gène codant pour la Cycline D2 ou la Cycline E1, permet de corriger le phénotype lié à l'absence de Cycline D1 ce qui atteste de cette redondance [Geng, Y. et al. Cell 97, 767-777 ; Carthon, B. C. et al. Mol Cell Biol 25, 1081-1088].

Basé sur l'étude de l'absence des Cyclines lors du développement [Ciemerych, M. A. & Sicinski, P. Oncogene 24, 2877-2898], une carence de la protection cellulaire par inhibition de l'apoptose grâce aux Cyclines D et E aura comme impact :

1- La Cycline D1 : sur

- les maladies neuro-dégénératives comme la perte de neurones dopaminergiques dans la maladie de Parkinson
- les insuffisances cardiaques et infarctus du myocarde associés à une mort cellulaire induite par stress oxidatif
- les difficultés à allaiter liées à un défaut de développement lobuloalvéolaire et/ou une apoptose exagérée des progéniteurs de la glande mammaire
- Une infertilité masculine associée à une fragilité à la mort cellulaire par stress environnemental des cellules

germinales

- Des troubles de la vue associés à une dégénérescence rétinienne

2- La Cycline D2 sur :

- Des troubles de l'équilibre et de la coordination des mouvements liés à une altération de l'homéostasie du cervelet
- La glycémie et l'apparition de diabète par défaut de sécrétion d'insuline par les cellules beta des ilôts de Langerhans
- Une infertilité féminine associée à une fragilité à la mort cellulaire par stress environnemental des ovaires
- Des troubles de la prostate associés à une perte de l'homéostasie de cet organe avec l'âge

3- La Cycline D3 sur :

- des problèmes infectieux par carence de la réponse immunitaire ou par perte de la réponse adaptative avec le temps
- des réactions hyper-allergiques ou des symptômes asthmatiques
- des troubles hémophiles
- des troubles respiratoires par défaut de sécrétion de surfactant ou par perte du réservoir de cellules Club (aussi appelées cellules de Clara)

4- La Cycline E1 sur :

- Des troubles cardiaques

5- La Cycline E2 sur :

- Une infertilité masculine

## Revendications

**1.** Méthode de pronostic *in vitro* de la susceptibilité à un stress environnemental d'un individu, la méthode comprenant

a. une étape de détermination de la séquence d'initiation de la traduction, ou séquence KOZAK, du gène *Ccnd1,* à partir d'un échantillon biologique dudit individu, et la comparaison avec une séquence correspondante de référence pour identifier des mutations,

b. une étape de quantification de l'expression de la protéine codée par ledit gène dont la séquence KOZAK a été séquencée à l'étape précédente, et la comparaison avec le niveau d'expression de ladite protéine issue d'un échantillon de référence,

de sorte que si la séquence KOZAK comprend une mutation et que le niveau d'expression de la protéine est modifié par rapport au niveau de référence, ledit individu sera plus susceptible de développer une pathologie liée à un stress environnemental.

**2.** Méthode de pronostic *in vitro* selon la revendication 1, dans laquelle si le niveau d'expression de la protéine est plus élevé que le niveau d'expression de référence, ledit individu aura plus de chance de développer une tumeur résistante aux chimiothérapies.

**3.** Méthode de pronostic *in vitro* selon la revendication 1, dans laquelle si le niveau d'expression de la protéine est moins élevé que le niveau d'expression de référence, ledit individu aura plus de chance de développer une maladie neurodégénérative, une maladie cardiaque ou une infertilité.

**4.** Méthode de pronostic in vitro selon l'une quelconque des revendications 1 à 3, dans laquelle la séquence de référence de la séquence KOZAK :

- du gène *Ccnd1* comprend la séquence suivante 5'-AGA GCC CCA GCC ATG GAA CAC CAG CTC -3' (SEQ ID NO : 1),
- .

**5.** Méthode de pronostic in vitro selon l'une quelconque des revendications 1 à 4, dans laquelle la quantification de l'expression de la protéine codée par ledit gène dont la séquence KOZAK a été séquencée est réalisée par FRET.

**6.** Méthode de pronostic *in vitro* de la résistance à une chimiothérapie d'une tumeur, ladite méthode comprenant une étape de détection d'une mutation dans la séquence KOZAK du gène *Ccnd1,* ladite mutation étant une substitution C->G en position -7 par rapport au codon ATG de la séquence SEQ ID NO : 1.

**7.** Utilisation d'une composition comprenant une paire d'oligonucléotides permettant le séquençage de la séquence KOZAK du gène codant la protéine Cycline D1,
dans le cadre du pronostic de résistance à une chimiothérapie d'une tumeur développée par un individu.

**8.** Utilisation selon la revendication 7, où ladite paire d'oligonucléiotides est la paire suivante : SEQ ID NO : 38 et 39.

**9.** Composition comprenant au moins un ARN interférant dirigé contre le gène *Ccnd1,* codant la protéine Cycline D1, pour son utilisation dans le cadre du traitement d'une tumeur résistante à une chimiothérapie, les cellules de ladite tumeur comprenant une mutation dans la séquence KOZAK du gène *Ccnd1,* ladite mutation étant une substitution C->G en position -7 par rapport au codon ATG de la séquence SEQ ID NO : 1.

**10.** Composition pour son utilisation selon la revendication 9, en combinaison avec une chimiothérapie, notamment l'un quelconque des composés suivants seul ou en combinaison : cisplatine, oxaliplatine, paclitaxel, irinotecan (SN38) et 5-FluoroUracil.

**11.** Composition pour son utilisation selon la revendication 9 ou 10, ladite composition consitant en l'un au moins des siRNA de séquence SEQ ID NO : 44, SEQ ID NO : 45 et SEQ ID NO : 46.

**Patentansprüche**

**1.** In-vitro-Prognoseverfahren für die Anfälligkeit eines Individuums für einen umweltbedingten Stress, wobei das Verfahren Folgendes umfasst:

a. einen Schritt des Bestimmens der Sequenz zur Einleitung der Translation, oder KOZAK-Sequenz, des Gens *Ccnd1,* ausgehend von einer biologischen Probe dieses Individuums, sowie des Vergleichens mit einer entsprechenden Referenzsequenz, um Mutationen zu erkennen,
b. einen Schritt des Quantifizierens der Expression des Proteins, das von dem Gen codiert wird, dessen KOZAK-Sequenz im vorhergehenden Schritt sequenziert wurde, sowie des Vergleichens mit dem Expressionsniveau des gleichen Proteins, das aus der Referenzprobe stammt,

derart, dass das Individuum anfälliger dafür sein wird, eine Pathologie zu entwickeln, die mit einem umweltbedingten Stress in Verbindung steht, wenn die KOZAK-Sequenz eine Mutation umfasst und das Expressionsniveau des Proteins gegenüber dem Referenzniveau verändert ist.

**2.** *In-vitro*-Prognoseverfahren nach Anspruch 1, wobei das Individuum mit höherer Wahrscheinlichkeit einen Tumor entwickeln wird, der gegen Chemotherapien resistent ist, wenn das Expressionsniveau des Proteins höher als das Referenz-Expressionsniveau ist.

**3.** In-vitro-Prognoseverfahren nach Anspruch 1, wobei das Individuum mit höherer Wahrscheinlichkeit eine neurodegenerative Erkrankung, eine Herzerkrankung oder eine Unfruchtbarkeit entwickeln wird, wenn das Expressionsniveau des Proteins niedriger als das Referenz-Expressionsniveau ist.

**4.** In-vitro-Prognoseverfahren nach einem beliebigen der Ansprüche 1 bis 3, wobei die Referenzsequenz der KOZAK-Sequenz:

• des Gens *Ccnd1* die folgende Sequenz 5'-AGA GCC CCA GCC ATG GAA CAC CAG CTC -3' (SEQ ID Nr.: 1) umfasst.

**5.** In-vitro-Prognoseverfahren nach einem beliebigen der Ansprüche 1 bis 4, wobei die Quantifizierung der Expression des Proteins, das von dem Gen codiert wird, dessen KOZAK-Sequenz sequenziert wurde, mittels FRET erfolgt.

6. In-vitro-Prognoseverfahren für die Resistenz eines Tumors gegen eine Chemotherapie, wobei das Verfahren einen Schritt des Nachweisens einer Mutation in der KOZAK-Sequenz des Gens *Ccnd1* umfasst, wobei es sich bei dieser Mutation um eine C->G-Substitution an der Position -7 handelt, unter Bezugnahme auf das ATG-Codon der Sequenz SEQ ID Nr.: 1.

7. Verwendung einer Zusammensetzung, die ein Paar von Oligonukleotiden umfasst, das eine Sequenzierung der KOZAK-Sequenz des Gens ermöglicht, das für das Protein Cyclin D1 codiert,
im Rahmen der Prognose bezüglich der Resistenz eines Tumors, den ein Individuum entwickelt hat, gegen eine Chemotherapie.

8. Verwendung nach Anspruch 7, wobei es sich bei dem Paar von Oligonukleotiden um das folgende Paar handelt: SEQ ID Nr.: 38 und 39.

9. Zusammensetzung, die mindestens eine interferierende RNA umfasst, die gegen das Gen *Ccnd1* gerichtet ist, das für das Protein Cyclin D1 codiert, zur Verwendung derselben im Rahmen der Behandlung eines Tumors, der gegen eine Chemotherapie resistent ist, wobei die Zellen dieses Tumors eine Mutation in der KOZAK-Sequenz des Gens *Ccnd1* umfassen, wobei es sich bei der Mutation um eine C -> G-Substitution an der Position -7 handelt, unter Bezugnahme auf das ATG-Codon der Sequenz SEQ ID Nr.: 1.

10. Zusammensetzung zur Verwendung derselben nach Anspruch 9, in Kombination mit einer Chemotherapie, insbesondere einer beliebigen der folgenden Verbindungen allein oder in Kombination: Cisplatin, Oxaliplatin, Paclitaxel, Irinotecan (SN38) und 5-Fluoruracil.

11. Zusammensetzung zur Verwendung derselben nach Anspruch 9 oder 10, wobei die Zusammensetzung aus mindestens einer der siRNA mit den Sequenzen SEQ ID Nr.: 44, SEQ ID Nr.: 45 und SEQ ID Nr.: 46 besteht.

## Claims

1. *In vitro* method for prognosis of the susceptibility to environmental stress of an individual, the method comprising :

   a. a step of determining the translation initiation sequence, or KOZAK sequence, of *Ccnd1* gene, from a biological sample of said individual and comparing with the corresponding reference sequence in order to identify mutations,
   b. a step of quantifying the expression of the protein coded by said gene the KOZAK sequence of which was determined in the previous step, and comparing with the expression level of the protein obtained from a reference sample,

   such that if the KOZAK sequence comprises a mutation and the expression level of the protein is modified relative to the reference level, said individual will be likelier to develop a pathology related to an environmental stress.

2. *In vitro* prognosis method according to claim 1, wherein if the expression level of the protein is higher than the reference expression level, said individual will have a greater chance of developing a tumor resistant to chemotherapy.

3. *In vitro* prognosis method according to claim 1, wherein if the expression level of the protein is not as high as the reference expression level, said individual will have a greater chance of developing a neurodegenerative disease, a cardiac disease or infertility.

4. *In vitro* prognosis method according to anyone of claims 1 to 3, wherein the reference sequence of the KOZAK sequence

   - of the *Ccnd1* gene comprises the following sequence 5'-AGA GCC CCA GCC ATG GAA CAC CAG CTC -3' (SEQ ID NO : 1).

5. *In vitro* prognosis method according to anyone of claims 1 to 4, wherein the quantification of the expression of the protein coded by said gene whose KOZAK sequence was sequenced is carried out by FRET.

6. *In vitro* method for prognosis of the resistance to chemotherapy of a tumor, said method comprising a step for

detecting a mutation in the KOZAK sequence of the *Ccnd1* gene, said mutation being a C->G substitution in position -7 relative to the ATG codon of the sequence SEQ ID NO: 1.

**7.** Use of a composition comprising a pair of oligonucleotides allowing sequencing of the KOZAK sequence of the gene coding the Cyclin D1, for the prognosis of resistance to chemotherapy of a tumor developed by an individual.

**8.** Use according to claim 7, Wherein the pair of oligonucleotides is the following pair SEQ ID NO: 38 and SEQ ID NO: 39.

**9.** Composition comprising at least one interfering RNA directed against the Ccnd1 gene, coding the cyclin D1 protein, for its use for the treatment of a chemotherapy-resistant tumor obtained from an individual, the cells of said tumor comprising a mutation in the KOZAK sequence of the Ccnd1 gene, said mutation being a C->G substitution in position -7 relative to the ATG codon of sequence SEQ DI NO: 1.

**10.** Composition for its use according to claim 9, in combination with a chemotherapy, in particular anyone of the following compounds alone or in combination: cisplatin, oxaliplatin, paclitaxel, irinotecan (SN38) and 5-FluoroUracil.

**11.** Composition for its use according to claim 9 or 10, said composition consisting of at least one of the siRNA of sequence SEQ ID NO: 44, SEQ ID NO: 45 and SEQ ID NO: to 46.

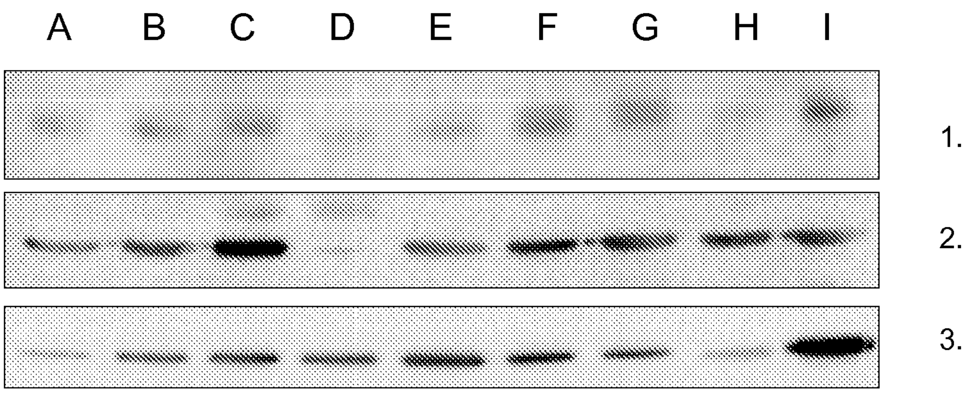


**Figure 1**

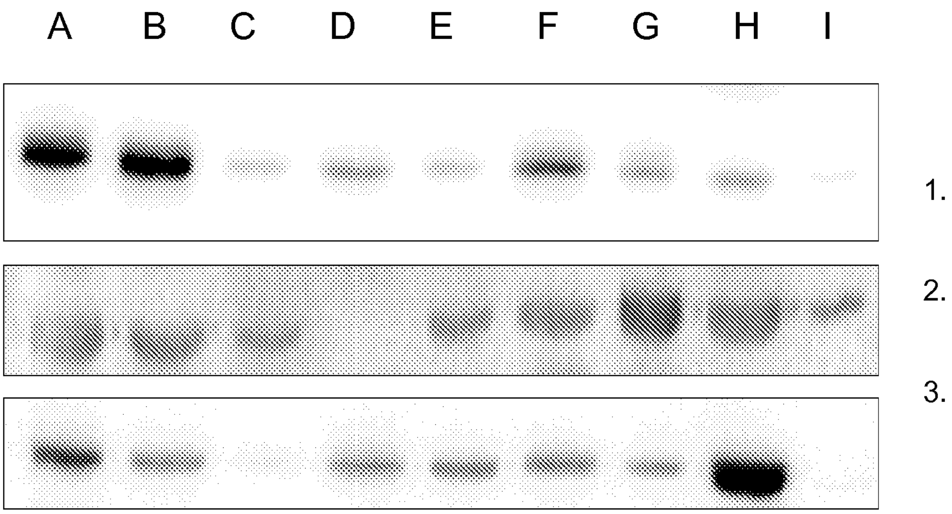


**Figure 2**

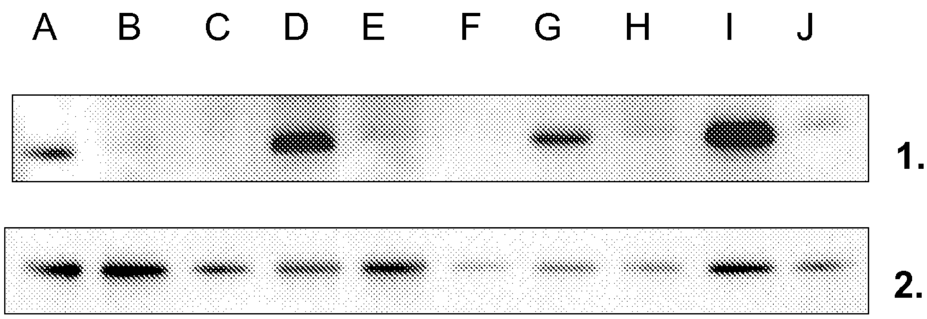


**Figure 3**

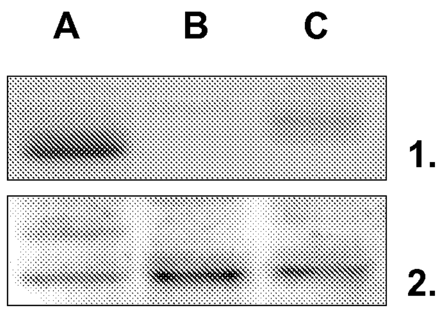


Figure 4

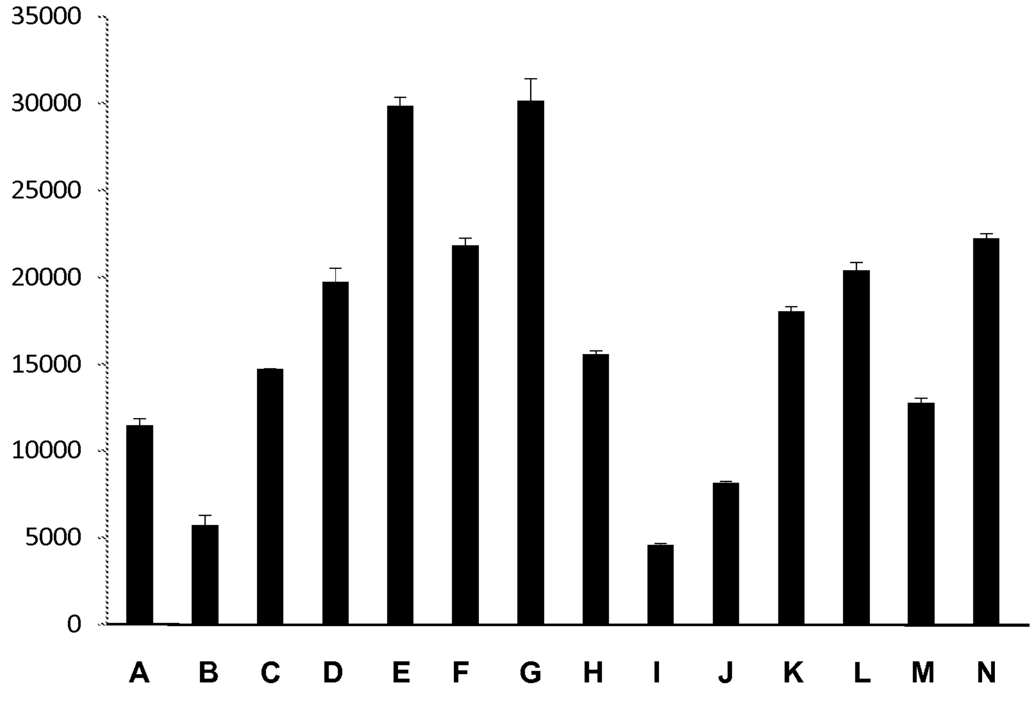


Figure 5A

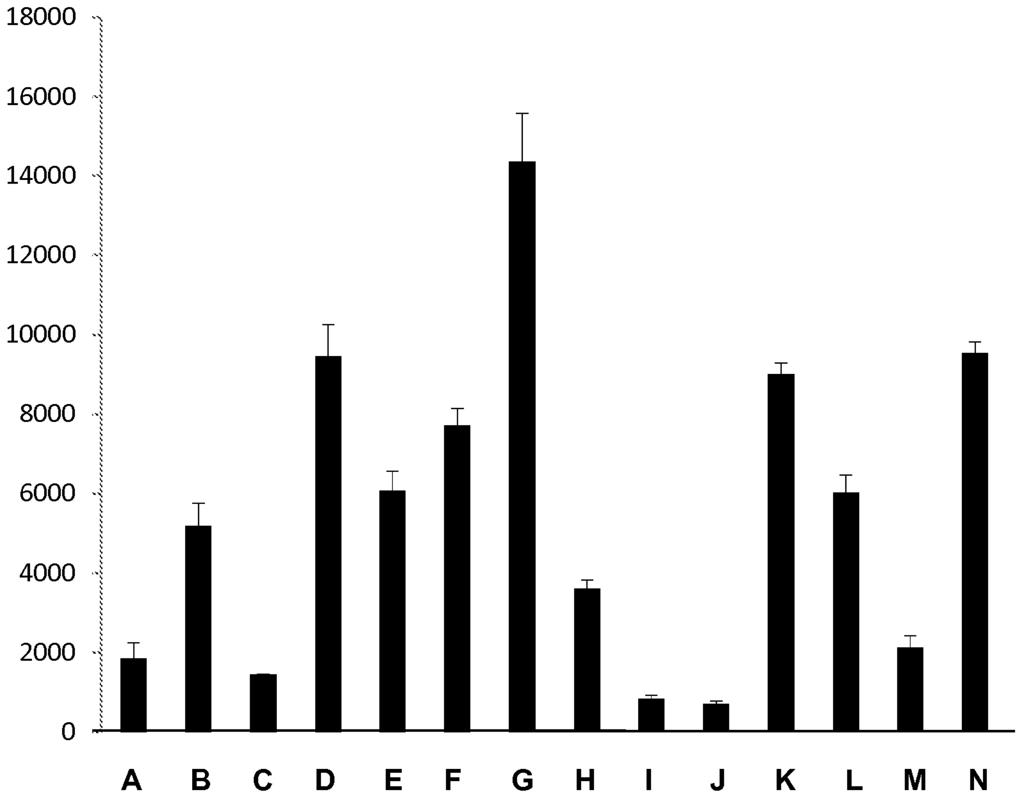


Figure 5B

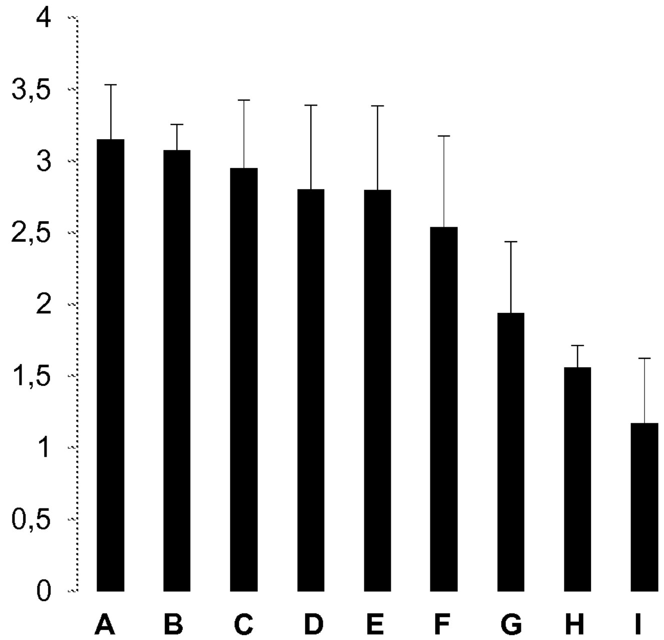


Figure 6

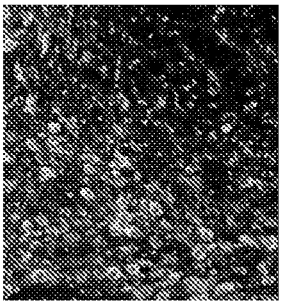

Figure 7A

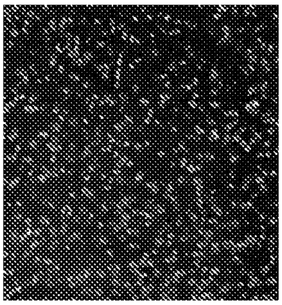

Figure 7C

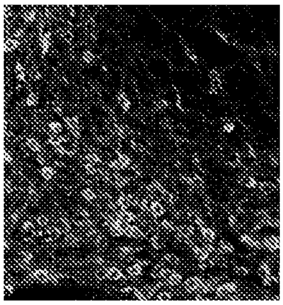

Figure 7E

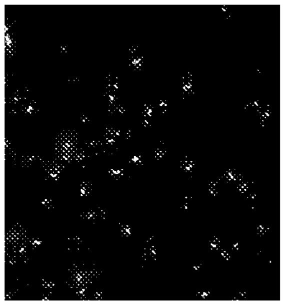

Figure 7G

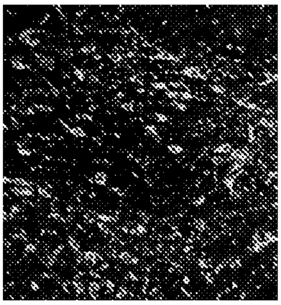

Figure 7B

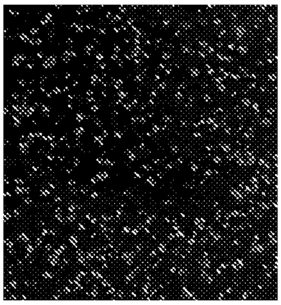

Figure 7D

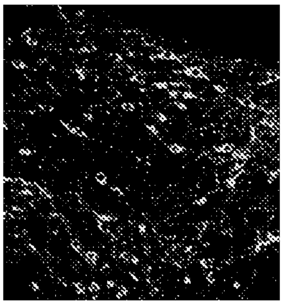

Figure 7F

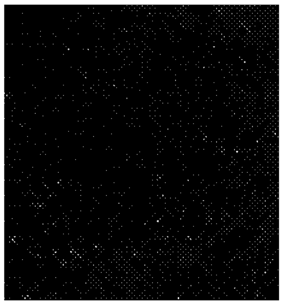

Figure 7H

Figures 7

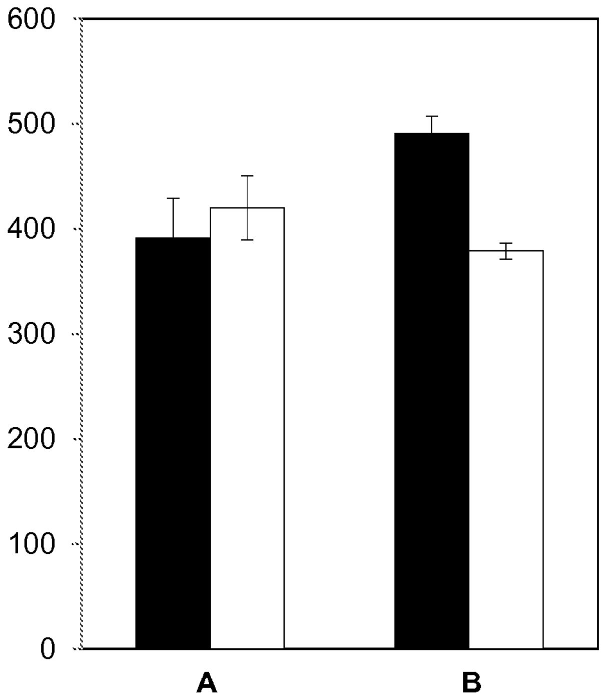


Figure 8

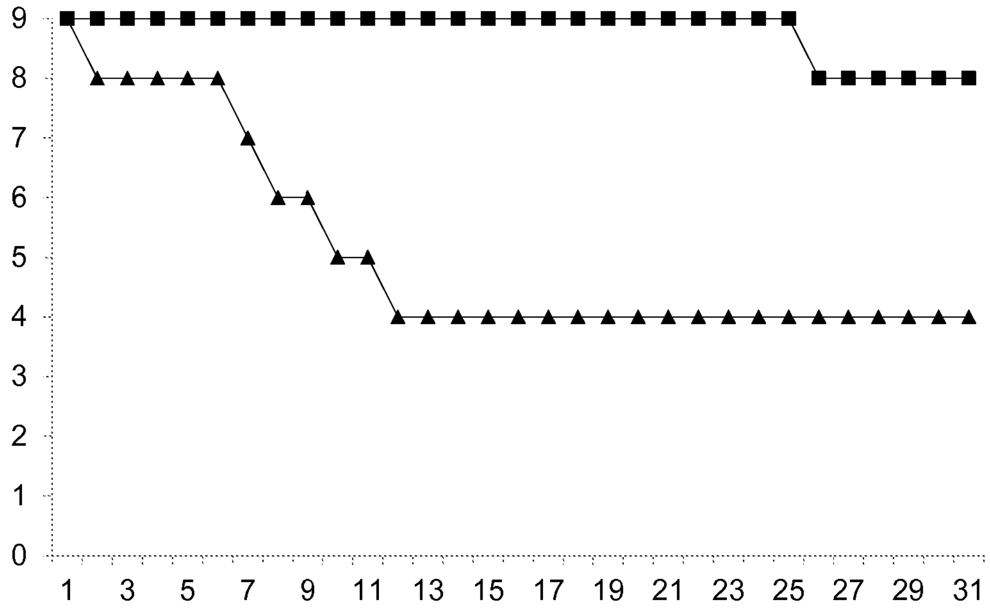


Figure 9

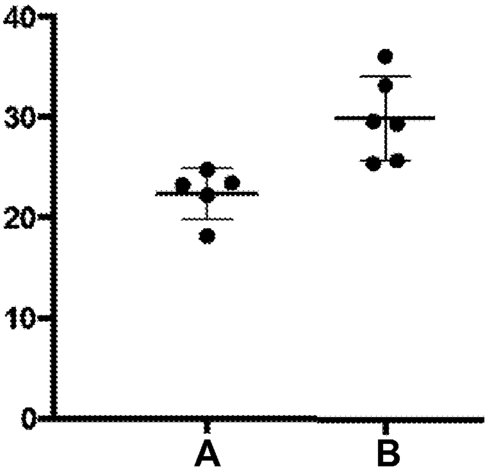


Figure 10

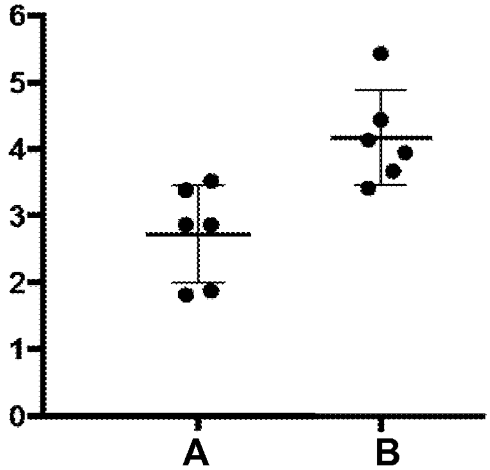


Figure 11

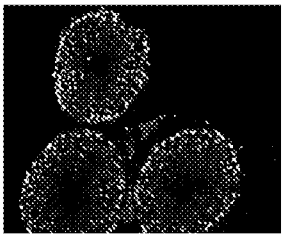
Figure 12A

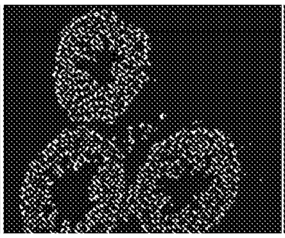
Figure 12C

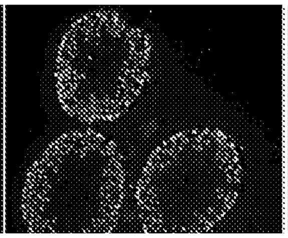
Figure 12E

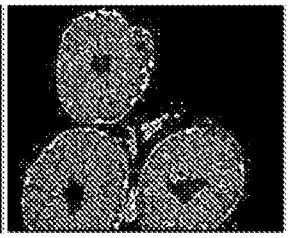
Figure 12G

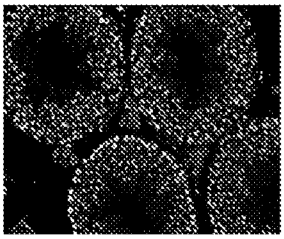
Figure 12B

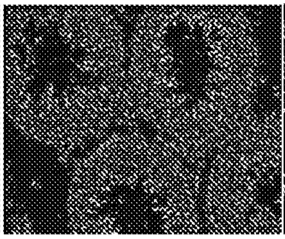
Figure 12D

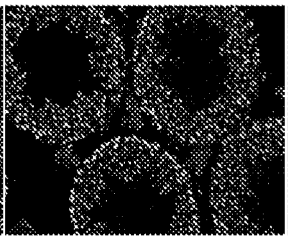
Figure 12F

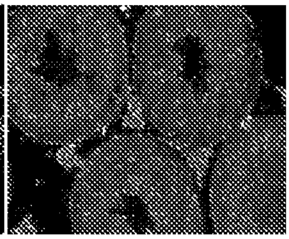
Figure 12H

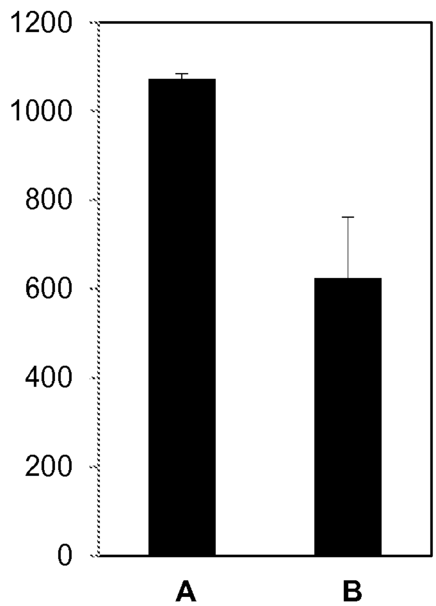

Figure 13

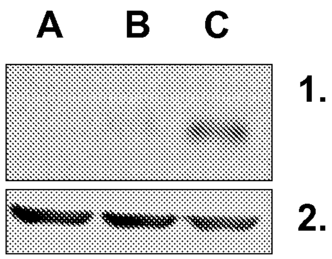


Figure 14

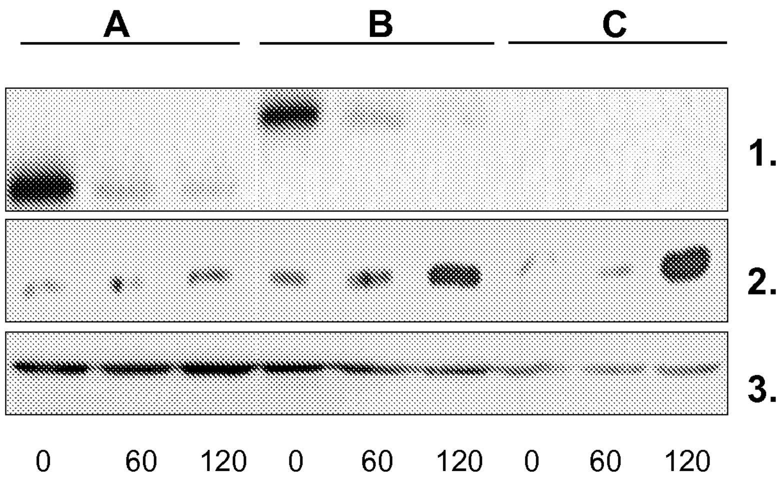


Figure 15

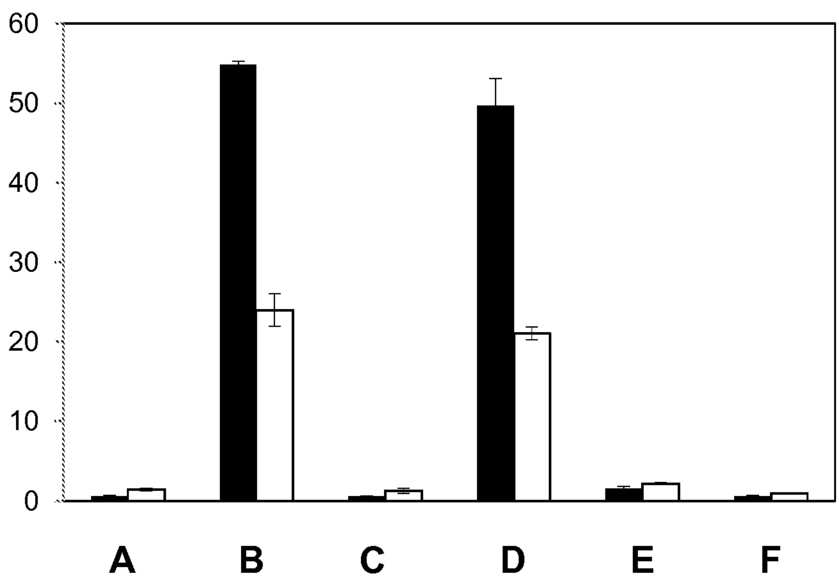


Figure 16

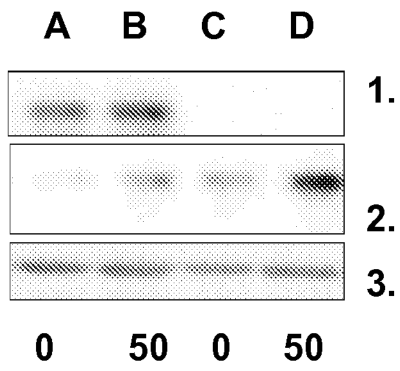


Figure 17

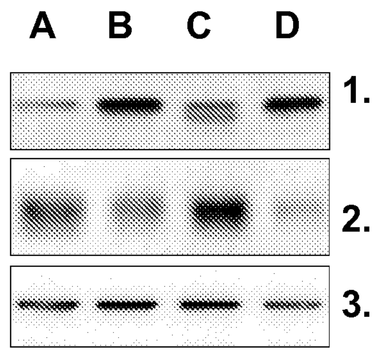


Figure 18

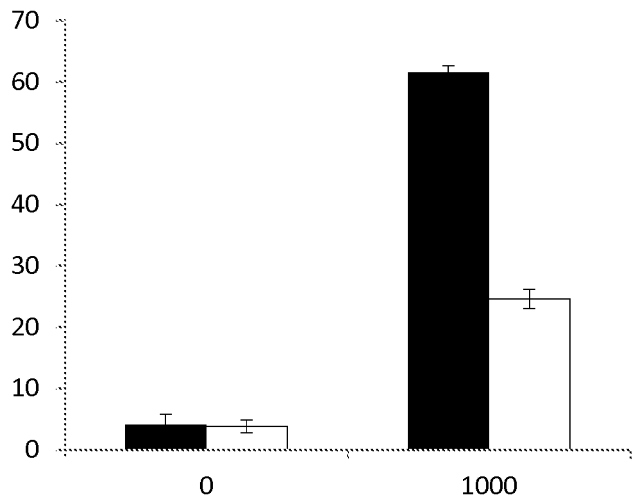


Figure 19

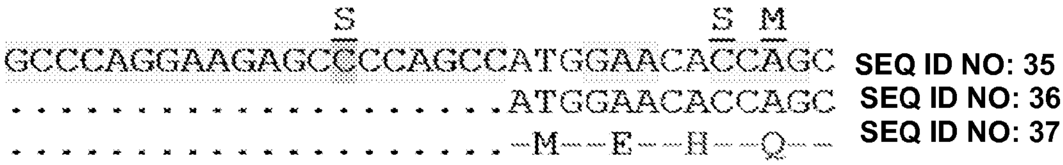


**Figure 20**

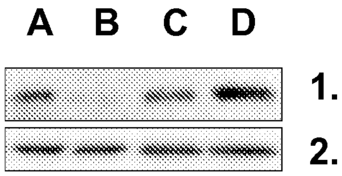


**Figure 21**

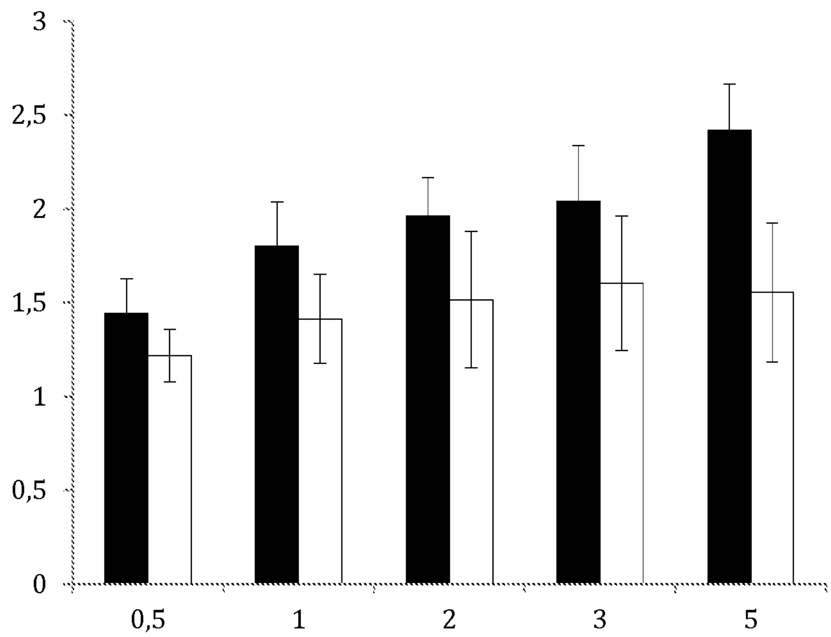


**Figure 22**

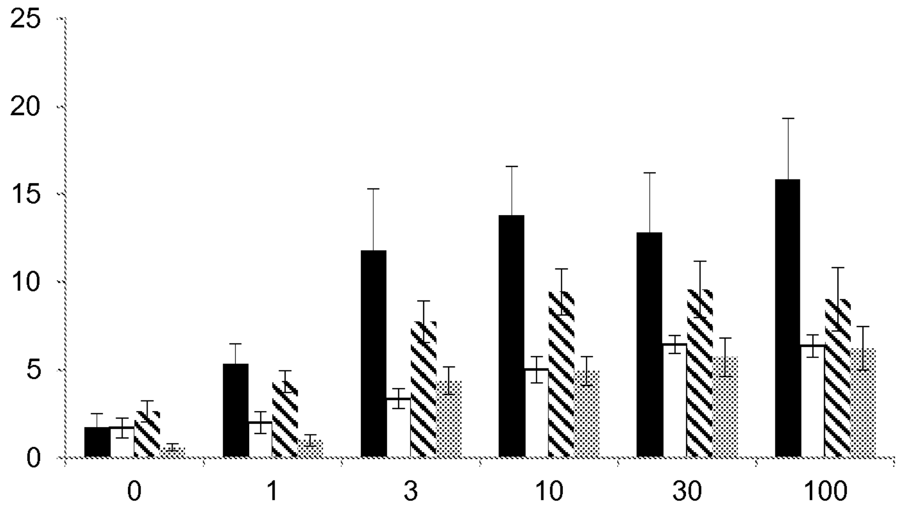


Figure 23

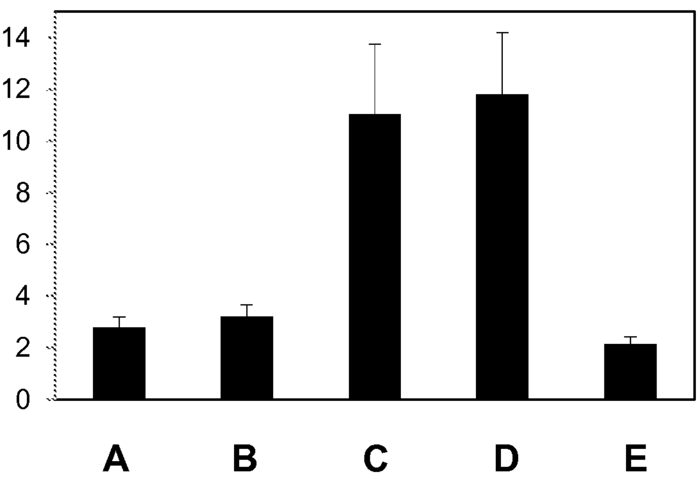


Figure 24

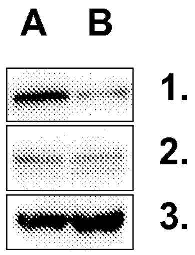


Figure 25

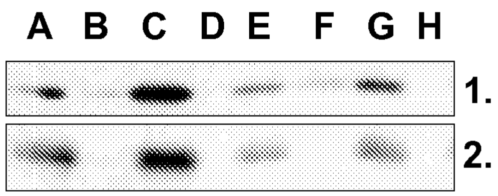


Figure 26

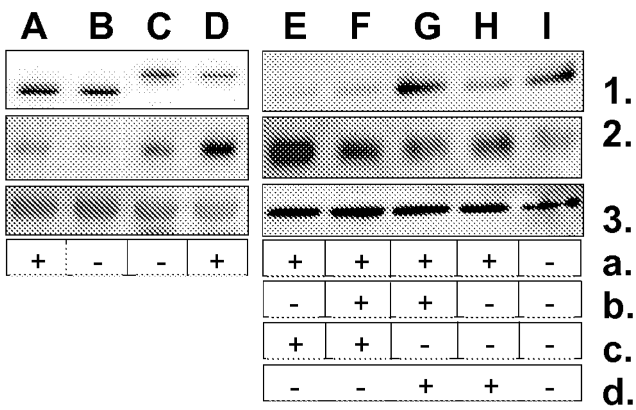


Figure 27

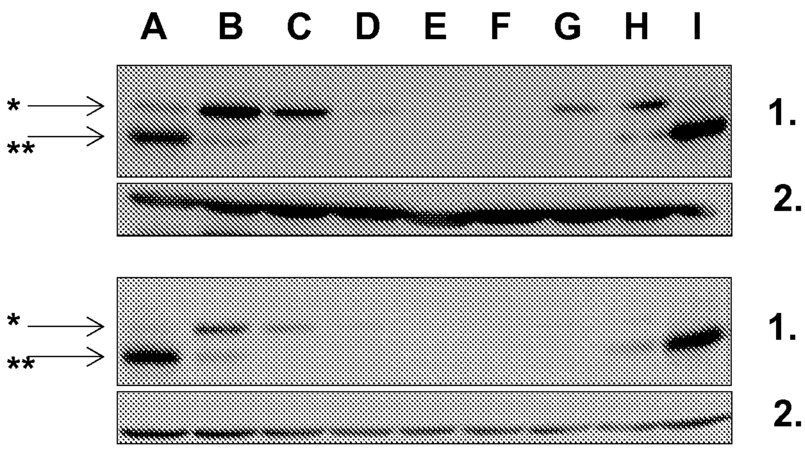


Figure 28

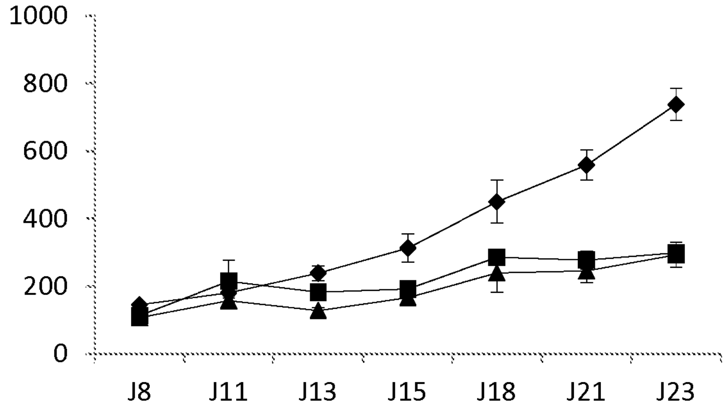


Figure 29

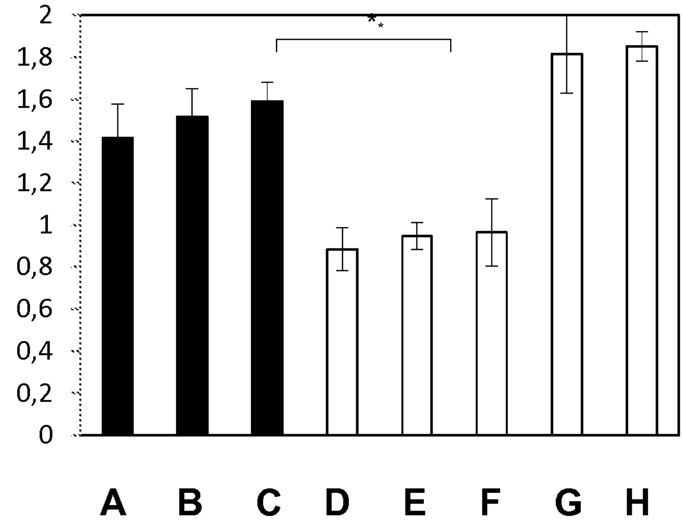


Figure 30

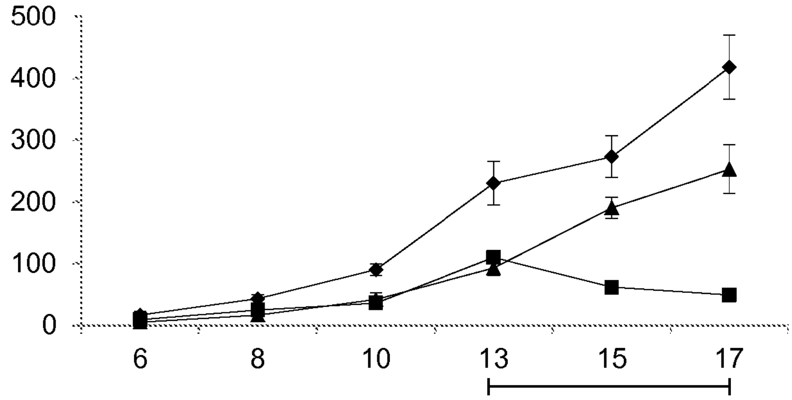


**Figure 31**

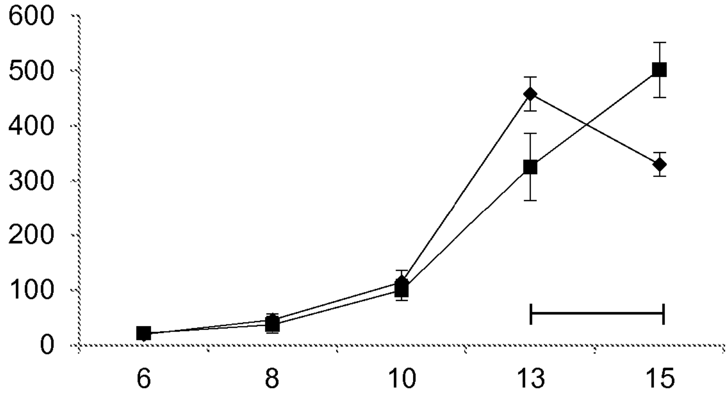


**Figure 32**

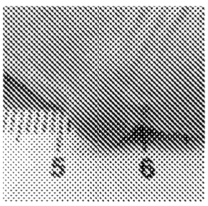


**Figure 33A**

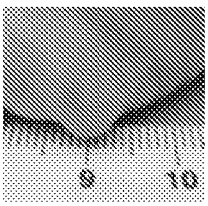


**Figure 33B**

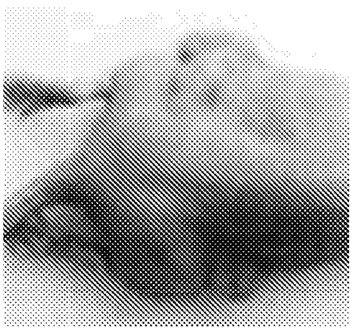

**Figure 33C**

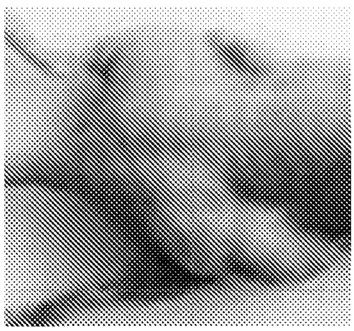

**Figure 33D**

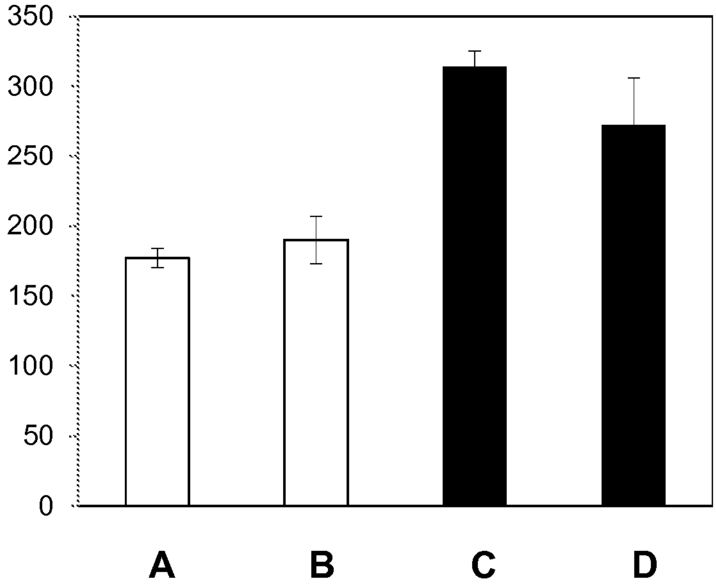


Figure 34

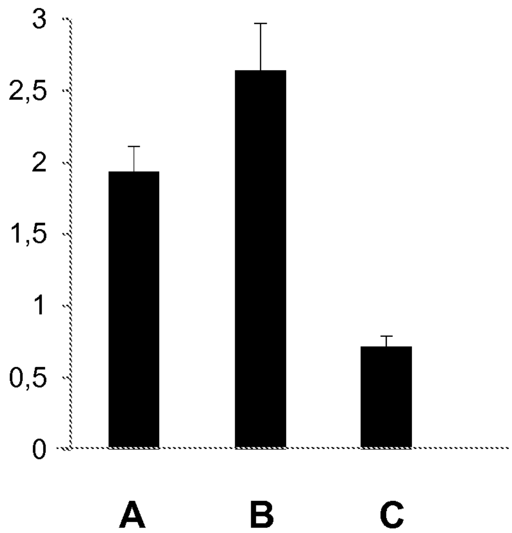


Figure 35

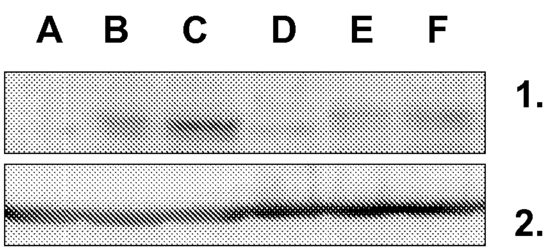


Figure 36

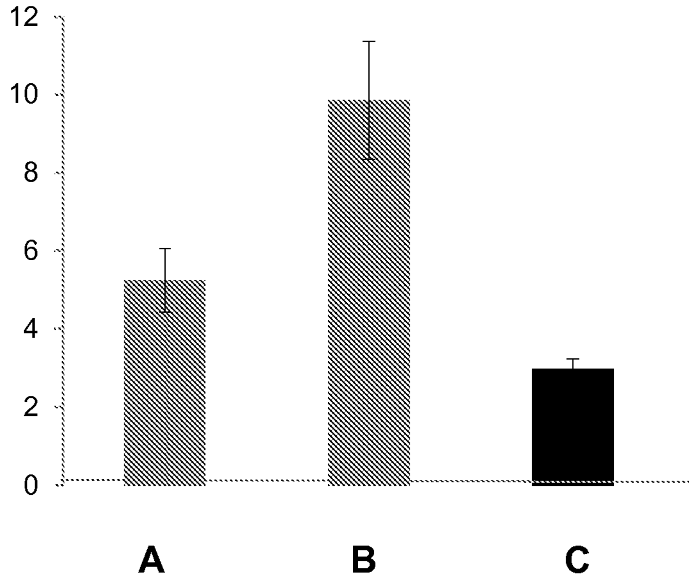


Figure 37

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 7091348 B2


### Littérature non-brevet citée dans la description


- **BIENVENU, F. et al.** *Nature,* 2010, vol. 463 (7279), 374-8
- **ARES-SANTOS, S. et al.** *Neuropsychopharmacology,* 2014, vol. 39 (5), 1066-80
- **MALKI, S. et al.** *EMBO J,* 2005, vol. 24 (10), 1798-809
- **BIENVENU et al.** *Nature,* 2010, vol. 463 (7279), 374-8
- **GENG, Y. et al.** *Cell,* vol. 97, 767-777
- **CARTHON, B. C. et al.** *Mol Cell Biol,* vol. 25, 1081-1088
- **CIEMERYCH, M. A. ; SICINSKI, P.** *Oncogene,* vol. 24, 2877-2898